(19) Europäisches Patentamt
European Patent Office
Office européen des brevets

(11) **EP 3 546 935 B1**

(12) **EUROPEAN PATENT SPECIFICATION**

(45) Date of publication and mention
of the grant of the patent:
**23.12.2020 Bulletin 2020/52**

(51) Int Cl.:
***G01N 33/487*** *(2006.01)*     ***G01N 33/50*** *(2006.01)*

(21) Application number: **18465611.4**

(22) Date of filing: **24.10.2018**

(54) **METHOD FOR IN VITRO DETECTION OF THE PROARRHYTHMOGENIC RISK OF A DRUG CANDIDATE ON HUMAN INDUCED PLURIPOTENT STEM CELL-DERIVED CARDIOMYOCYTES (HIPSC-CM)**

VERFAHREN ZUR IN-VITRO-DETEKTION DES PROARRHYTHMOGENEN RISIKOS EINES ARZNEIMITTELKANDIDATEN FÜR KARDIOMYOZYTEN, DIE VON MENSCHLICHEN INDUZIERTEN PLURIPOTENTEN STAMMZELLEN ABGELEITET SIND (HIPSC-CM)

PROCÉDÉ DE DÉTECTION IN VITRO DU RISQUE PRO-ARRYTHMOGÈNE D'UN MÉDICAMENT CANDIDAT SUR DES CARDIOMYOCYTES ISSUS DE CELLULES SOUCHES PLURIPOTENTES INDUITES (HIPSC-CM)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(43) Date of publication of application:
**02.10.2019 Bulletin 2019/40**

(73) Proprietor: **Universitatea din Bucuresti**
**050107 Bucharest (RO)**

(72) Inventors:
• **AMUZESCU, Bogdan**
  **Bucuresti (RO)**
• **RADU, Beatrice Mihaela**
  **050713 Bucuresti (RO)**
• **MIHAILESCU, Dan Florin**
  **Chitila, Jud. Ilfov (RO)**
• **MANN, Stefan Alexander**
  **50226 Frechen (DE)**

(74) Representative: **Vasilescu, Raluca**
**Cabinet M. Oproiu**
**Patent and Trademark Attorneys**
**42, Popa Savu Street**
**Sector 1, P.O. Box 2-229**
**011434 Bucharest (RO)**

(56) References cited:
**WO-A2-2017/172825**

• **OLAF SCHEEL ET AL: "Action Potential Characterization of Human Induced Pluripotent Stem Cell-Derived Cardiomyocytes Using Automated Patch-Clamp Technology", ASSAY AND DRUG DEVELOPMENT TECHNOLOGIES, vol. 12, no. 8, October 2014 (2014-10), pages 457-469, XP055583395, US ISSN: 1540-658X, DOI: 10.1089/adt.2014.601**
• **ALBERTAS I. UNDROVINAS ET AL: "Ranolazine Improves Abnormal Repolarization and Contraction in Left Ventricular Myocytes of Dogs with Heart Failure by Inhibiting Late Sodium Current", JOURNAL OF CARDIOVASCULAR ELECTROPHYSIOLOGY., vol. 17, no. s1, May 2006 (2006-05), pages S169-S177, XP055583533, US ISSN: 1045-3873, DOI: 10.1111/j.1540-8167.2006.00401.x**
• **Alison Obergrussberger ET AL: "Automated Patch Clamp Recordings of Human Stem Cell-Derived Cardiomyocytes" In: "Stem Cell-Derived Models in Toxicology", 26 November 2016 (2016-11-26), Springer New York, New York, NY, XP055583376, ISSN: 1557-2153 ISBN: 978-1-4939-6661-5 pages 57-82, DOI: 10.1007/978-1-4939-6661-5_4, * page 58 - page 60 * * figures 1-3 ***

**(Cont. next page)**

- Gary R Mirams ET AL: "Application of cardiac electrophysiology simulations to pro-arrhythmic safety testing", British Journal of Pharmacology, November 2012 (2012-11), pages 932-945, XP055583501, Oxford, UK DOI: 10.1111/j.1476-5381.2012.02020.x Retrieved from the Internet: URL:https://bpspubs.onlinelibrary.wiley.com/doi/pdf/10.1111/j.1476-5381.2012.02020.x

- THOMAS O'HARA ET AL: "Simulation of the Undiseased Human Cardiac Ventricular Action Potential: Model Formulation and Experimental Validation", PLOS COMPUTATIONAL BIOLOGY, vol. 7, no. 5, 26 May 2011 (2011-05-26), page e1002061, XP055583603, DOI: 10.1371/journal.pcbi.1002061

**Description**

FIELD OF THE INVENTION

The invention is related to cardiac safety testing, particularly in the field of detecting proarrhythmogenic risk of a drug candidate using an *in vitro* method on human induced pluripotent stem cell-derived cardiomyocytes (hiPSC-CM).

BACKGROUND OF THE INVENTION

In the field of cardiac safety testing there is a constant evolution from empirical tests based on experimental evidence to detailed in-depth approaches that offer an abundance of specific quantitative data using *in vitro* methods.

One of the hot areas of cardiac safety testing is the propensity of medicinal products to induce ventricular cardiac arrhythmia. Older assessment methods were based on prolongation of QT interval, representing the time between the start of the Q wave and the end of the T wave in the heart's electrical cycle as measured through electrocardiography (ECG), thus using *in vivo* testing of the candidate drugs.

Whereas many experts in the field and big pharma companies consider direct *in vivo* testing of new drugs on conscious animals via ECG telemetry or on healthy human volunteers as "golden standard", it can be scientifically proved that effects recorded via methods such as "corrected QT interval prolongation" are often non-specific and devoid of predictive power, let aside the important health-related risks and hazards for healthy human volunteers directly subjected to new pharmacological compounds with insufficiently characterized or completely unknown toxicological profile (1).

Over time, a large number of *in vitro* preparations have been successfully used to evaluate different proarrhythmogenic effects of candidate drugs, starting with the artificially perfused isolated heart model developed by Oskar Langendorff and its subsequent modern variants ["working heart"], continuing with "myocardial wedge" preparations, isolated papillary muscle or isolated Purkinje fibers approached by two-microelectrode voltage-clamp, considered the most sensitive to pharmacological effects due high ion channel surface densities required to ensure high velocities of action potential (AP) propagation (2).

Although still highly used in pharmacology assays, these methods and preparations feature a series of disadvantages and inconveniences, such as large amounts of residual tissue acting like reservoirs or "sinks" for drugs, lack of reproducibility and specificity, as well as the need to use large groups of experimental animals that are sacrificed, incurring high prices and low economic effectiveness. There are also differences concerning pharmacological responses and arrhythmogenesis mechanisms between human and animal myocardial tissue, therefore results obtained using animal preparations are provisional and require further confirmation by trials in humans.

For these reasons, a simplified *in vitro* test was developed, namely screening of the effects of medicinal products on hERG (*human ether-à-go-go related gene*) delayed rectifier K$^+$ channels, which represent the substrate of rapid delayed rectifier K$^+$ current ($I_{Kr}$), the main repolarizing ion current in cardiomyocytes, and further using specific highly sensitive voltage-clamp protocols applied to hERG channels expressed in heterologous systems such as HEK293 cells (human embryo kidney - a diploid cell line). This simplified test gained in popularity and became the method of election for cardiac safety drug testing due to simplicity and ease of automation (3).

This success is a consequence of the fact that most drug-induced arrhythmias consist in torsades-de-pointes (TdP) or polymorphic ventricular tachycardia, resulting in turn from synchronization of early afterdepolarizations (EAD), a frequent proarrhythmogenic condition at single-cardiomyocyte level, and most afterdepolarizations occur as a result of hERG channels block.

Most *in vivo* and *in vitro* cardiac safety tests, measuring QT interval prolongation on electrocardiogram or its equivalent in myocardial tissue approached with multielectrode arrays (MEA), belong to the "hERG-centric" paradigm, together with direct hERG inhibition *in vitro* tests.

The success of the "hERG-centric" cardiac safety testing paradigm has been tremendous: since its adoption in 2005 via ICH (International Conference on Harmonization of Technical Requirements for Registration of Pharmaceuticals for Human Use) standards S7B (for non-clinical *in vivo* and *in vitro* tests) and E14 (for clinical trials) not a single case of withdrawal from markets of a drug due to lethal arrhythmia accidents occurred.

However, the "hERG-centric" paradigm is far from being perfect, as revealed recently by the MICE (Multiple Ion Channel Effects) study performed at the drug screening company ChanTest (4). This study demonstrated convincingly that hERG screening alone (or its QT prolongation substitutes) is not 100% predictive for torsadogenic effects. A good counterexample is verapamil, a hERG-blocking compound that is not torsadogenic, because it also blocks L-type Ca$^{2+}$ channels with similar potency, and these two blocking effects of an outward and of an inward ion current balance and compensate each other.

This led to proposal of a new cardiac safety testing paradigm, the CiPA paradigm (Comprehensive in vitro Proarrhythmia Assay), first discussed on July 23, 2013 at a "think tank" meeting organized by FDA (United States Food and Drug Administration) at Silver Spring, MD, and endorsed by a number of drug regulatory agencies and high-profile research institutes (5).

It is known that the presence of a drug candidate may lead to the inhibition of certain ion currents and subsequent distortion of the shape of the externally paced action potential (AP), possibly with appearance of proarrhythmogenic effects such as early or delayed afterdepolarizations.

CiPA proposes an in-depth mechanistic cardiac safety approach based on three steps: 1. study of inhibitory effects of a drug candidate on multiple human cardiac ion currents (corresponding to ion channels) in heterologous expression systems, preferably by automated patch-clamp; 2. use of these inhibition data applied to an advanced human ventricular cardiomyocyte electrophysiology mathematical model, such as the O'Hara-Rudy2011 model (6) to predict proarrhythmogenic events such as early or delayed afterdepolarizations; 3. experimental validation of these predictions via pharmacology experiments on human induced pluripotent stem cell-derived cardiomyocytes (hiPSC-CM) using different methods. The CiPA paradigm may rescue some drug compounds rejected previously based on hERG inhibition criteria, but which are in fact not torsadogenic.

Some progress on the long road of implementing the CiPA paradigm into current practice has been achieved for both experimental and *in silico* steps (7): new optimized voltage-clamp protocols have been defined (8, 9), Makov gating models for hERG channels have been included in the O'Hara-Rudy model with multiple drug-bound states (10) and temperature dependence (11), and the net charge representing the integral over an entire AP of several inward and outward ion currents has been assessed as a predictor of torsadogenic risk (12).

Several approaches have been proposed for the third step of CiPA, including studies on cardiomyocyte layers approached with multi-electrode arrays (MEA) to measure extracellular local field potentials (13-16) or impedance changes (17-21) or optical recordings on human induced pluripotent stem cell-derived cardiomyocytes hiPSC-CM loaded with voltage-sensitive or calcium-sensitive fluorescent dyes (22-28).

Another method to test pharmacological effects on hiPSC-CM is the whole-cell patch-clamp approach, with either manual or automated equipment. Complex assays can be applied to hiPSC-CM that combine well-defined voltage-clamp protocols to assess the effects of a drug on specific cardiac ion current components with current-clamp protocols to test the effects of the same drug on externally-triggered AP shape, as described for example in Scheel et al. 2014. Action potential Characterization of Human Induced Pluripotent Stem Cell-Derived Cardiomyocytes Using Automated patch-Clamp Tehnology", Assay and Drug Development Technologies, vol. 12, no. 8, October 2014, pp. 457-469, XP055583395, US.

Disadvantages of prior art

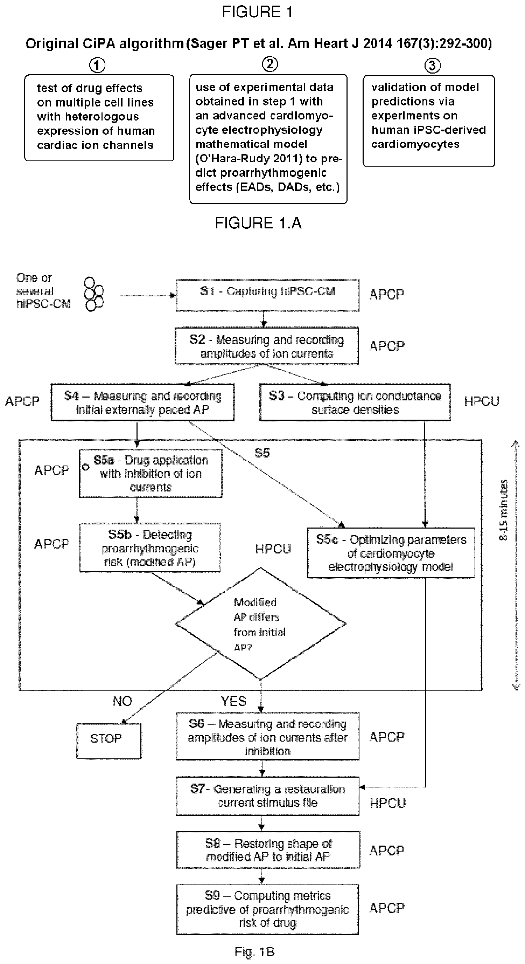

CiPA paradigm, representing the closest prior art for the invention (see Figure 1a), has some disadvantages:

i) Heterologous expression cell lines often express only the main transmembrane subunit of a cardiac ion channel but not the accessory (ancillary) subunits. The accessory subunits exert strong influence on biophysical as well as pharmacological properties, or cellular viability, sealing suitability, or stability in time of the ion current. Thus the results of the assessment of proarrhythmogenic effects of drug candidates may not be satisfactory because of the impossibility to take into consideration all factors that have influence on pharmacological properties.

ii) Although methods known from prior art, such as MEA, are easy to implement and susceptible to automation and high throughput, the information they provide is limited and often equivalent to QT prolongation obtained in *in vivo* trials, hence devoid of specificity and predictive power. Only global effects on spontaneous pacemaking frequency, overall AP shape and parameters at cardiomyocyte population level, conduction velocity and arrhythmogenesis via reentry mechanisms can be studied by such methods. Detailed characterization of specific inhibitory effects of drug candidates on distinct cardiac ion currents or control of transmembrane potential in measured cells are often impossible.

iii) Other disadvantage of prior art refers to the weaknesses of optical methods used on human induced pluripotent stem cell-derived cardiomyocytes hiPSC-CM loaded with voltage-sensitive or calcium-sensitive fluorescent dyes (22-28) regarding the lack of knowledge of resting potential of the cardiomyocyte (or maximal diastolic potential for pacemaking cells), which is a very important parameter (29), high signal-to-noise ratios, and cell toxicity phenomena upon intense light exposure.

iv) Other optical methods, particularly those developed by Molecular Devices, based on proprietary FLIPR® (fluorescence imaging plate reader) platforms, allow measurement of specific inhibitory effects of drug on $K^+$ channels by quantitating $Rb^+$ or $Tl^+$ inflow; although adequate for high-throughput screening, these methods do not achieve an adequate control of transmembrane voltage and are not capable of detailed characterization of effects.

v) Yet other disadvantage of the closest prior art is the length of the detection of the proarrhythmogenic risk, as various steps of the paradigm may take place at different times, not necessarily in the same day.

Problem solved by the invention

The problem to be solved by the invention is how to detect the proarrhythmogenic risk of a drug candidate using a rapid method that is able to process a large quantity of mechanistic data regarding the inhibitory effect of the drug candidate over the cardiomyocytes using a type of cardiomyocyte cells that express not only the main transmembrane subunit of a cardiac ion currents but also the accessory (ancillary) subunits.

SUMMARY OF THE INVENTION

In order to solve the problem of the invention, the inventors conceived a method of detecting *in vitro* the proarrhythmogenic risk of a drug candidate where, by use of a high-performance automated patch-clamp platform APCP able to carry out experimental measurements and recordings of at least five human ventricular ion currents and of an externally paced action potential AP performed on human induced pluripotent stem cell-derived cardiomyocytes hiPSC-CM and by use of a computerized algorithm running on a high-performance computer unit HPCU for rapid parameter optimization of an advanced cardiomyocyte electrophysiology mathematical model to accurately reproduce the shape of said externally paced action potential (AP), the following one-shot sequence of steps is carried out on said human induced pluripotent stem cell-derived cardiomyocytes hiPSC-CM:

Step1 - Capturing on a microfluidic chip of one or several human induced pluripotent stem cell-derived cardiomyocytes hiPSC-CM from a suspension, forming of gigaseals, followed by achieving of whole-cell configuration by β-escin perforation, which allows to stabilize the externally paced action potential AP shape over a duration generally between 10 and 20 minutes;

Step 2 - Separating, recording, and measuring peaks or steady-state amplitudes of the at least five ion currents by applying to said one or several human induced pluripotent stem cell-derived cardiomyocytes hiPSC-CM approached in β-escin perforated whole-cell patch-clamp of a series of well-defined voltage-clamp protocols;

Step 3 - Computing based on automated analysis of recordings using the series of said voltage-clamp protocols of ion conductance surface densities for each of the at least five said ion currents and of scaling factors for ion conductance surface densities of each of said at least five ion currents relative to default values of said advanced cardiomyocyte electrophysiology mathematical model;

Step 4 - Measuring and recording an initial externally paced action potential AP of said one or several human induced pluripotent stem cell-derived cardiomyocytes hiPSC-CM by applying a standard current-clamp protocol with a single sweep and with predetermined current stimuli.

Step 5

- 5a Applying said drug candidate at a given concentration on one or several human induced pluripotent stem cell-derived cardiomyocytes hiPSC-CM fulfilling 2 conditions, namely having a resting potential of of < -50mV and a having a ventricular-like initial externally paced action potential AP with an Action Potential Duration ratio APD50/APD90 > 70% , said application of the drug candidate being for a pre-determined period of time, preferably 8 to 15 minutes, and more preferably 10 minutes, and simultaneously, during the same pre-determined period of time:

- 5b- Measuring and recording the modified externally paced action potential AP under the effect of said drug candidate by applying the same current-clamp protocol as for determining initial externally paced action potential AP, periodically at fixed intervals of time, preferably one-minute, and detecting proarrhythmogenic effects of said candidate drug on the initial externally paced action potential AP shape, such as early or delayed afterdepolarizations;

- 5c- Rapid optimization of parameters of said advanced cardiomyocyte electrophysiology mathematical model using the said scaling factors for ion conductance surface densities of each of said at least five ion currents in order to accurately reproduce the shape of said externally paced initial action potential AP on human induced pluripotent stem cell-derived cardiomyocytes hiPSC-CM having as result a model-generated shape of said externally paced initial action potential AP;

Step 6 - Measuring and recording amplitudes of the at least five ion currents inhibited after application of said drug candidate using the same series of voltage-clamp protocols as in step 2, comparing them with initial amplitudes in the absence of said drug candidate and calculating percentages of inhibition for each of them;

Step 7 - Generating a restauration current stimulus file representing the sum of all ion currents inhibited by the candidate drug with reversed sign and the initial predetermined stimuli, to be used further to restore the initial externally paced action potential AP shape in the one or several human induced pluripotent stem cell-derived cardiomyocytes hiPSC-CM subjected to the action of the drug candidate by running a simulation with said advanced cardiomyocyte electrophysiology mathematical model with previously optimized parameters and the percentages of inhibition calculated in the previous step or known in advance from previous experiments;

Step 8 - Restoring the modified externally paced action potential AP to its initial shape, recorded before the application of said drug candidate by applying of the restauration current stimulus file using a dynamic-clamp protocol to the same one human induced pluripotent stem cell-derived cardiomyocyte hiPSC-CM on which the drug candidate was applied;

Step 9 - Using the calculated percentages of inhibition to compute metrics predictive of proarrhythmogenic risk of said drug candidate such as the net charge integrated over an action potential carried by the sum of several inward and outward ion currents.

Advantages of the invention

The main advantages of this invention are the following:

i) The rapidity of the method, as compared with prior art. In less than 30 minutes the detection of the proarrhythmogenic risk of the drug candidate is carried out;

ii) The use of human induced pluripotent stem cell-derived cardiomyocytes hiPSC-CM during all steps of the method carried out on a high-performance automated patch-clamp platform (APCP), as compared with prior art where only some steps of the paradigm are carried out on such cardiomyocytes, allows tests on a cellular preparation expressing not only the main transmembrane subunit of a cardiac ion channel but also the accessory (ancillary) subunits of said cardiac ion channels providing ion currents used by the method;

iii) A better accuracy of detection due to the optimization of the externally paced action potential (AP) and due to the precision of the calculation of the ion conductance surface densities;

iv) The possibility to validate the method of detection of the proarrhythmogenic risk of the drug candidate;

v) The use of β-escin allows to stabilize the shape of the initial externally paced action potential (AP) and the shape of the modified action potential (AP) after application of the drug candidate, said preventing the run-down phenomenon accompanied by progressive externally paced action potential (AP) shortening.

BRIEF DESCRIPTION OF THE DRAWINGS

Figure 1a. Diagram of experimental and computation steps according to CiPA paradigm;

Figure 1 b. Diagram of the method according to the invention;

Figure 2. Voltage-clamp protocol for measuring peak voltage-dependent Na$^+$ current amplitude (Nav_standard_v2_MC defined in CytoLogic);

Figure 3. Voltage-clamp protocol for separating L-type Ca$^{2+}$ current $I_{CaL}$ from voltage-dependent Na$^+$ current $I_{Na}$ (CaVNaVSeparation_MC defined in CytoLogic);

Figure 4. Voltage-clamp protocol for measuring transient outward current $I_{to}$, delayed rectifier K$^+$ current $I_{Kr}+I_{Ks}$ (mainly $I_{Kr}$), and "funny" hyperpolarization-activated current $I_f$ (hERG_Standard_m120_m50_MC defined in CytoLogic);

Figure 5. (Table 1) Changes and settings applied to the original O'Hara-Rudy2011 C++ script in order to use it for rapid model parameter optimization relative to experimental data during performance of the experimental steps of the method on the automated patch-clamp platform (APCP);

Figure 6. AP shape obtained by running the modified O'Hara-Rudy2011 model by numeric integration with default parameters for subendocardial, subepicardial, and midmyocardial (M type) human ventricular cardiomyocytes, at physiological temperature ($PT$ = 37°C = 310 K)(default temperature in original O'Hara-Rudy 2011 model) vs. room temperature ($RT$ = 25°C = 298 K);

Figure 7. Effects on AP shape of varying the scaling factors of several ion conductance densities from 0.3 to 3 times the default values for M type in the O'Hara-Rudy 2011 model;

Figure 8. (Table 2) Based on analysis shown in previous figure, classification of ion currents and corresponding ion conductance surface densities into components with marked effects on the shape of AP and duration vs. components with reduced effects;

Figure 9. Results of a computerized parameter optimization algorithm for the modified O'Hara-Rudy 2011 model to fit experimental data, with optimization of different parameters applied in sequential order and computations run on a single processor;

Figure 10. Flowchart of a parameter optimization algorithm using several rounds of massively parallel computations;

Figure 11. Results of parameter optimization of the modified O'Hara-Rudy 2011 model using a sequential monoprocessor algorithm to fit experimental data obtained in automated patch-clamp experiments on Cor.4U® cardiomyocytes;

Figure 12. (Table 3) Comparison of decay rates (in % change/min) between classical "ruptured" whole-cell patch-clamp and β-escin-perforated patch-clamp applied in automated patch-clamp experiments with the CytoPatch™2 platform on Cor.4U® hiPSC-CM, using a complex assay that combines multiple voltage-clamp and current-clamp protocols;

Figure 13. Comparison of AP plateau run-down and triangulation over 10 min. with current-clamp recordings using a standard protocol: 3 current stimuli of 0.5 ms 2 nA at 3-s interval, first stimulus at 500 ms from start, applied at 1-min, intervals (overlapped traces) in a Cor.4U® cardiomyocyte perforated by classical "ruptured" whole-cell automated patch-clamp (upper panel) with situation when the cardiomyocyte is approached via β-escin-perforated patch-clamp (lower panel).

Figure 14. A complete run of the method on a Cor.4U® cardiomyocyte approached by automated patch-clamp with a CytoPatch™2 platform in the β-escin-perforated configuration, including AP recordings in initial conditions, after nifedipine application, and during the dynamic-clamp protocol using the restauration current stimulus file.

List of abbreviations (in alphabetic order)

AP - Externally-paced Action Potential

APCP - Automated patch-clamp platform

APD - [Externally-paced] Action Potential Duration

APD50- [Externally-paced] Action Potential Duration corresponding to 50% repolarization of the membrane of the cardiomyocyte

APD90- [Externally-paced] Action Potential Duration corresponding to 90% repolarization of the membrane of the cardiomyocyte

CiPA - Comprehensive in vitro Proarrhythmia Assay

DAD - Delayed afterdepolarizations

DF - Driving force

EAD - Early afterdepolarizations

ECG - Electrocardiography

hiPSC-CM - Human induced pluripotent stem cell-derived cardiomyocytes

HPCU - High-performance computer unit

$I_{CaL}$ - L-type Ca2+ current

$I_f$ - Hyperpolarization-activated (funny) current

$I_{K1}$ - Weak inward rectifier K+ current

$I_{Kr}$ - Rapid delayed rectifier K+ current

$I_{Kr}+I_{Ks}$ - Delayed rectifier K+ current

$I_{Ks}$ - Slow delayed rectifier K+ current

$I_{Na}$ - Voltage-dependent Na+ current

$I_{to}$ - Transient outward current

MEA - Multielectrode array

MICE - Multiple Ion Channel Effects

$C_m$ - Membrane capacitance

$P_a$ - Activation probability

$P_{ssi}$ - Steady-state inactivation probability

$PT$ - Physiological temperature

QT - Time interval between the start of the Q wave and the end of the T wave on electrocardiogram

$R_a$ - Access resistance

$R_m$ - Membrane resistance

RP - Resting Potential

$RT$ - Room temperature

scgcal - Scaling factor for conductance density for the L-type $Ca^{2+}$ current

scgf - Scaling factor for conductance density for the hyperpolarization-activated (funny) current

scgkr - Scaling factor for conductance density for the rapid delayed rectifier $K^+$ current scgna - Scaling factor for conductance density for the $Na^+$ current

scgto - Scaling factor for conductance density for the transient outward current

TdP - Torsades-de-Pointes, the most frequent drug-induced arrhtyhmia

$V$off - Voltage offset

## DETAILED DESCRIPTION

The method according to the invention uses two types of equipment. The first is the high-performance automated patch-clamp platform APCP capable of performing usual types of protocols i.e. voltage-clamp protocols, current-clamp protocols, dynamic-clamp protocols, used in the invention to carry out experimental measurements and recordings. The second equipment is the high-performance computer unit HPCU for rapid parameter optimization of an advanced cardiomyocyte electrophysiology mathematical model using an algorithm running on said high-performance computer unit HPCU.

According to the invention, the automated patch-clamp-platform APCP measures and records values of at least five human cardiac ion currents, in short ion currents, and of the externally paced action potential AP. The selection of the ion currents may depend on various criteria, such as but not limited to their relevance to the particular drug candidate and the possibility to measure accurately the values thereof.

The method is conceived to take place in a one-shot sequence of steps, unlike the prior art methods where some steps of the known methods can take place with pauses between them.

The general flowchart of the method is illustrated in Figure 1b, in contrast with the general flowchart of the CiPA paradigm from the state-of-art, depicted in Figure 1a.

The method starts with the preparation steps for the microfluidic chip used by the automated patch-clamp platform: filling with external and internal solutions, priming, voltage offset adjustments and capacitive transient cancellations, measurement of pipette tip resistance on each channel.

Then, in the <u>first step</u> of the method carried out on the automated patch-clamp-platform APCP, one or several human induced pluripotent stem cell-derived cardiomyocytes hiPSC-CM are captured on the microfluidic chip from a suspension, and gigaseals are formed, followed by achievement of whole-cell configuration by β-escin perforation. It is preferable to achieve whole-cell configuration by β-escin perforation in order to stabilize the shape of the externally paced action potential (AP) over a duration generally between 10 and 20 minutes.

The general parameters of the equivalent electrical circuit (membrane capacitance - $C_m$, access resistance - $R_a$, membrane resistance - $R_m$, and voltage offset - $V_{off}$ in current-clamp mode) are monitored; fast and slow capacitive transients are cancelled and series resistance compensation can be applied.

For the β-escin perforated variant a good way to monitor perforation is to apply repeatedly a voltage-clamp protocol with a depolarizing step to elicit fast voltage-dependent Na$^+$ current $I_{Na}$, and to plot its peak amplitude at different moments of time.

Best results in term of seal quality and stability are obtained with a third generation automated patch-clamp platform, e.g. CytoPatch™2, which applies the proprietary Cytocentering® method (patent US20030153067A1) (30) based on small microfluidic chips containing quartz pipette tips with well-defined geometry, with inner tip diameters of 2 or 2.5 μm, surrounded by cytocentering orifices.

The inferior pole of a cell applied in suspension into the transport channel is aspired by the cytocentering orifice and placed tightly over the quartz pipette tip, enabling high quality and stability of gigaseals, using physiological external and internal solutions.

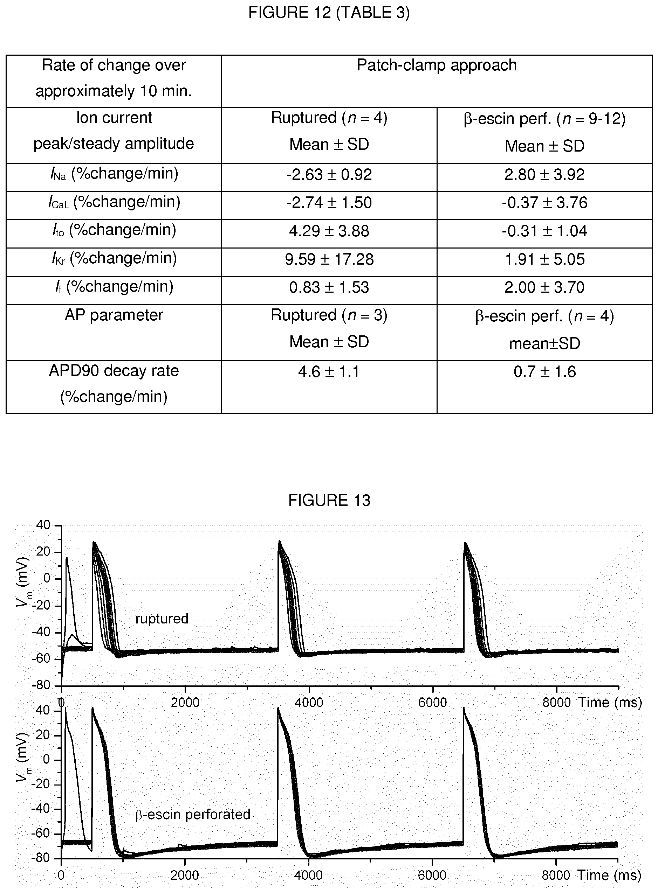

In one preferred embodiment β-escin perforated whole-cell patch-clamp on the one or several human induced pluripotent stem cell-derived cardiomyocytes hiPSC-CM is performed in order to stabilize initial externally paced action potential AP shape and initial peak or steady-state amplitudes of said at least five ion currents over a time interval of 10-20 min. in control conditions, preventing the run-down phenomenon accompanied by progressive shortening of said initial externally paced action potential AP shape that occurs in the classical "ruptured" whole-cell patch-clamp approach on said one or several human induced pluripotent stem cell-derived cardiomyocytes hiPSC-CM (Figure 12(Table 3) and Figure 13).

Subsequently, <u>in step 2</u> of the method, also carried out on the automated patch-clamp-platform APCP, after achieving the whole-cell configuration, a series of voltage-clamp protocols is applied to said one or several human induced pluripotent stem cell-derived cardiomyocytes hiPSC-CM in order to separate, record and measure steady-state amplitudes of the at least five ion currents, and said peak or steady-state amplitudes are measured and the results recorded.

In another preferred embodiment, used hereafter as a non-limiting example of the method, the following human ventricular ion currents are used:

- peak rapid voltage-dependent inward Na$^+$ current ($I_{Na}$);
- peak L-type inward Ca$^{2+}$ current ($I_{CaL}$);
- transient outward K$^+$ current ($I_{to}$);
- "funny" hyperpolarization-activated current ($I_f$);

- a sum of the rapid ($I_{Kr}$) and slow ($I_{Ks}$) components of the delayed rectifier $K^+$ current, but where usually $I_{Kr}$ is the dominant component in control conditions, where control conditions mean that no drug candidate was yet applied.

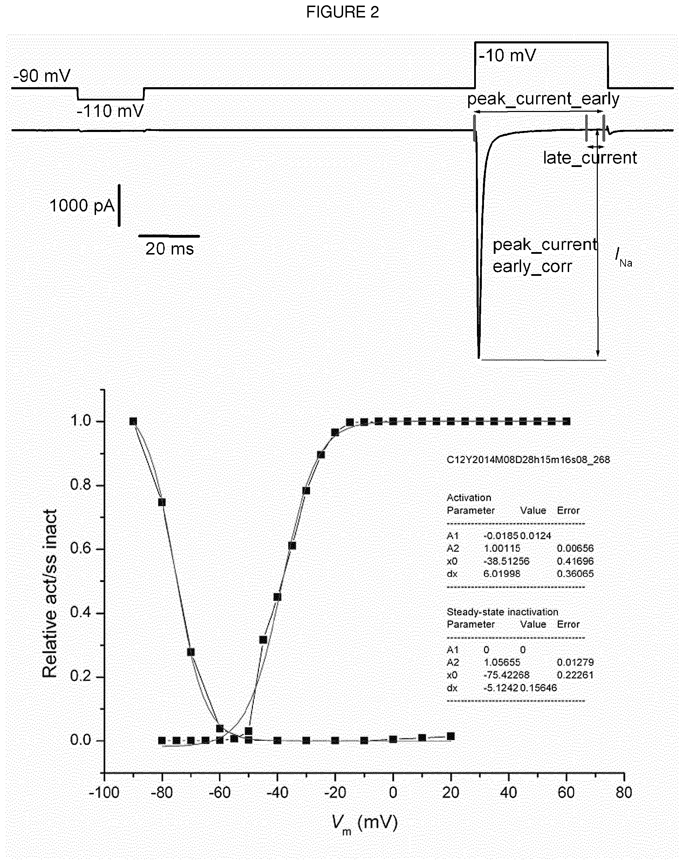

A first voltage-clamp protocol, illustrated in Figure 2, named Nav_standard_v2_MC as defined in the CytoLogic software that controls the CytoPatch™2 platform, and with the use of which the entire method may be carried out, activates $I_{Na}$ via a depolarizing step at -10 mV from a holding potantial of -90 mV.

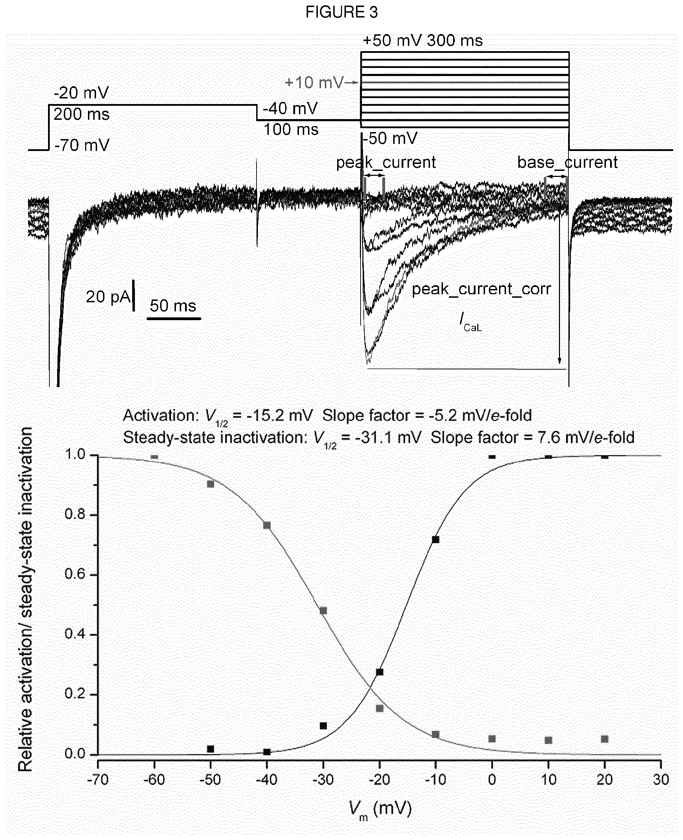

A second voltage-clamp protocol, illustrated in Figure 3 (CaVNaVSeparation_MC defined in CytoLogic) allows L-type $Ca^{2+}$ ion current $I_{CaL}$ separation from $I_{Na}$ based on different voltage dependencies of steady-state inactivation for these two currents (lower panels of Figure 2 and Figure 3).

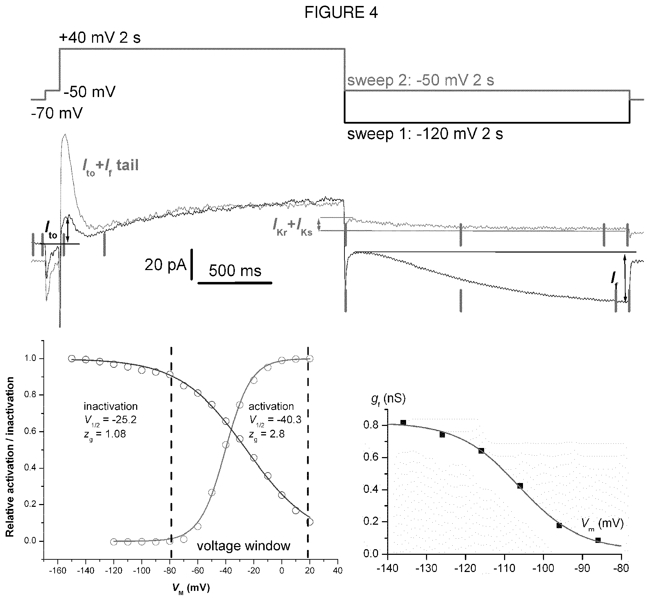

A third voltage-clamp protocol, illustrated in Figure 4 (hERG_Standard_m120_m50_MC defined in CytoLogic) derived from a standard hERG protocol by adding a supplementary first sweep with the second large voltage step at -120 mV, allows separation and measurement of multiple ion currents: transient outward $K^+$ current $I_{to}$ at the beginning of the first depolarizing step at +40 mV of the first sweep, the "funny" hyperpolarization-activated current $I_f$ activated gradually during the 2-s hyperpolarizing step of the first sweep, and the delayed rectifier $K^+$ current (a sum of rapid and slow components $I_{Kr} + I_{Ks}$, but where $I_{Kr}$ usually predominates and can be approximately estimated) measured as a small peak current at the beginning of the 2-s step at -50 mV of the second sweep, produced by rapid removal from inactivation of delayed rectifier $K^+$ channels at this potential, followed by a slow exponential deactivation with similar time constants for $I_{Kr}$ and $I_{Ks}$.

The above example with three voltage-clamp protocols shall not be considered as limiting, another number of voltage-clamp protocols fall within the scope of the invention as long as the purpose of separating, measuring and recording peak or steady-state amplitudes of the at least five ion currents is achieved.

In step 3 of the method, the recordings previously obtained by initial application of the voltage-clamp protocols are exported and sent to the high-performance computer unit HPCU in an easily readable file format (such as but not limited to Axon Text File - ATF) and automatically analyzed by said high-performance computer unit HPCU with a program, such as for example a C++ script that computes, based on automated analysis of said recordings, ion conductance surface densities for the at least five ion currents, such as for example for $I_{Na}$, $I_{CaL}$, $I_{to}$, $I_{Kr}$, $I_f$ using manually entered $C_m$ and $R_a$ values, by detecting peak current values between well-defined time intervals and base values measured as averages over other well-defined time intervals (limits of intervals used to measure peak and base current values marked with short vertical lines in Figures 2, 3, and 4), and applying a series of computations including voltage corrections for liquid junction potential and sometimes for voltage drops across access resistance, computation of driving force (DF) for each ion current component as difference between actual transmembrane voltage and Nernst equilibrium potential (or Goldmann-Hodgkin-Katz potential for $I_f$), obtaining ion conductance surface densities by dividing corrected peak (or steady for $I_f$) current amplitudes to DF and $C_m$, and supplementary correction by dividing these conductance densities to activation probabilities ($P_a$) and steady-state (removal from) inactivation probabilities ($P_{ssi}$) for each ion current using Boltzmann charge-voltage functions obtained from own experimental data (as shown in lower panels of Figures 2-4) or from literature for $I_{to}$ (32).

In addition to ion conductance surface densities, the same program, such as said C++ script computes scaling factors for said ion conductance surface densities for the at least five measured ion currents, such as for example for $I_{Na}$, $I_{CaL}$, $I_{to}$, $I_{Kr}$, $I_f$, relative to default values of said ion conductance surface densities of the advanced cardiomyocyte electrophysiology mathematical model.

In the same afore-mentioned embodiment of the invention the advanced cardiomyocyte electrophysiology mathematical model is O'Hara-Rudy 2011 human ventricular cardiomyocyte model and the scaling factors corresponding to the ion currents selected $I_{Na}$, $I_{CaL}$, $I_{to}$, $I_{Kr}$, $I_f$ are named scgna, scgcal, scgto, scgkr, and scgf, respectively. After completing the initial voltage-clamp protocols, the automated patch-clamp platform (APCP) switches to current-clamp mode for carrying out step 4 of the method, in which a standard current-clamp protocol is applied and the initially externally paced action potential (AP) is measured and recorded. Said standard current-clamp protocol is comprised of a single sweep lasting 9 s, and 3 brief current stimuli of 0.5 ms duration and 2 nA amplitude applied at 3-s intervals, the first stimulus being applied at 500 ms from start of recording.

If a current-clamped human induced pluripotent stem cell-derived cardiomyocyte (hiPSC-CM) features spontaneous pacemaking, this activity can be suppressed by applying continuous small hyperpolarizing currents (-10 or -20 pA), as shown in a previous own study (33).

If on one channel of the high-performance automated patch-clamp platform there is at least one human induced pluripotent stem cell-derived cardiomyocyte (hiPSC-CM) with good resting potential and nice ventricular-like initial externally paced action potential AP, the method is continued with steps 5-9. Good resting potential is defined by a value less than -50mV (< - 50mV), whereas nice ventricular-like initial externally paced action potential AP is considered when it has an APD50/APD90 ratio >70%, where APD stands for Action Potential Duration and the numbers 50, respectively 90 refer to the percentages of repolarization of 50% respectively 90% of the membrane of said cardiomyocyte. Thus the method is continued only for human induced pluripotent stem cell-derived cardiomyocytes hiPSC-CM that satisfy both above-mentioned conditions.

In step 5 of the method three different actions are carried out during a pre-determined period of time, preferably for 8 to 15 minutes, and more preferably for 10 minutes. The example of realization has been carried out for a pre-determined period of time of 10 minutes.

In sub-step 5a, on the automated patch-clamp platform APCP, the candidate drug at a certain concentration is applied for the pre-determined period of time, preferably for 8 to 15 minutes, and more preferably for 10 minutes. If the candidate drug at given concentration blocks or inhibits one or several ion currents, this will distort the shape of the initial externally paced action potential AP, possibly producing proarrhythmogenic effects such as early or delayed afterdepolarizations (EAD or DAD), which *per se* are an important indication of arrhythmogenic (torsadogenic) risk of the candidate drug.

In sub-step 5b measuring and recording of the modified values of the externally paced action potential AP is carried out on the automated patch-clamp platform APCP in current-clamp mode with the same series of protocols as used for measuring and recording initial externally paced action potential AP, said measurement and recording of the modified values occurring at fixed intervals of time, preferably 1-min.

Thus the detection of the proarrhythmogenic effects of the candidate drug is basically made in step 5b of the method and confirmed in the remainder of the steps.

If the candidate drug has no proarrhythmogenic effects, the measurement and recording carried out in sub-step 5b returns values of the externally paced action potential AP identical to the initial ones as measured and recorded in step 2, in which case the method is stopped. During the pre-determined period of time, preferably for 8 to 15 minutes, and more preferably for 10 minutes, while the candidate drug is applied to the one or several human induced pluripotent stem cell-derived cardiomyocytes hiPSC-CM and pharmacological effects of blocking or inhibition occur, the computerized algorithm running on the high-performance computer unit HPCU is applied in sub-step 5c for rapid optimization of parameters of the advanced cardiomyocyte electrophysiology mathematical model, such as modified O'Hara-Rudy 2011 model, to match the experimentally recorded series of the externally paced action potential AP with the standard stimulation protocol with the model-generated series of externally paced action potentials AP using the same stimulation protocol as in the experimental recording.

In step 6 of the method, the inhibitory effects of the candidate drug on each of the at least five ion currents are measured and recorded by the high-performance automated clamp platform APCP using the same series of voltage-clamp protocols as used in step 2, and the results of the measured and recorded peak or steady-state amplitudes are compared with the values of the initial corresponding amplitudes measured and recorded in the absence of said drug candidate. This step of the method ends with calculation of percentages of inhibition for each of the at least five ion currents.

The calculated percentages of inhibition may be compared, for validation purposes, with known values of percentages of inhibition of each of the at least five ion currents as resulted from other previous experiments that are different from present invention, such as for example experiments carried out on human cardiac ion channels expressed in heterologous systems.

Using either the calculated percentages of inhibition of the previous steps, or the known values of the percentages from other previous experiments, possibly also more complex state-dependent binding data or voltage shifts of activation or steady-state inactivation with more complex mathematical models including Markov models for different ion current components, e.g. a Markov model of hERG published in (10), in step 7 of the method, a restauration stimulus file is generated by the program running on the high-performance computer unit HPCU, preferably the C++ script, in predefined format readable by the software controlling the automated patch-clamp platform APCP by summation of the ion currents generated by the optimized model (read from an output file) with the estimated percentages of inhibition, with reversed sign, because stimulus current by convention has opposite sign to transmembrane current, and said restauration stimulus is added to the initial set of current stimuli of the standard current-clamp protocol (in the example the 3 current stimuli of 0.5 ms 2 nA amplitude at 3-s intervals, first stimulus at 500 ms).

This restauration stimulus file is applied in step 8 of the method to the same one or several human induced pluripotent stem cell-derived cardiomyocytes hiPSC-CM in a dynamic-clamp protocol on the automated patch-clamp platform APCP. The application of said restauration stimulus file has as effect restauration to its initial shape of the modified externally paced action potential AP shape distorted by the inhibition provoked by the candidate drug, by electronically supplying the ion currents that were suppressed by said candidate drug.

In the last step of the method, the 9th, the calculated percentages of inhibition of the previous steps, or the known values of the percentages from other previous experiments as used for the generation of the restauration stimulus file are used to compute on the high-performance computer unit HPCU used in the invention or on another similar equipment metrics predictive of proarrhythmogenic risk of said drug candidate such as the net charge integrated over an action potential carried by the sum of several inward and outward ion currents (12).

According to another embodiment of the invention the optimization of one or several parameters of said advanced cardiomyocyte electrophysiology mathematical model such as but not limiting to scaling factors of conductance surface densities, temperature, is performed in a sequential, parallel, or combined manner on the high-performance computer unit HPCU, taking as optimality criterion the sum of squared differences between the experimentally recorded initial externally paced action potential AP and the model-generated shape of said externally paced initial action potential AP and using some or all the scaling factors previously computed.

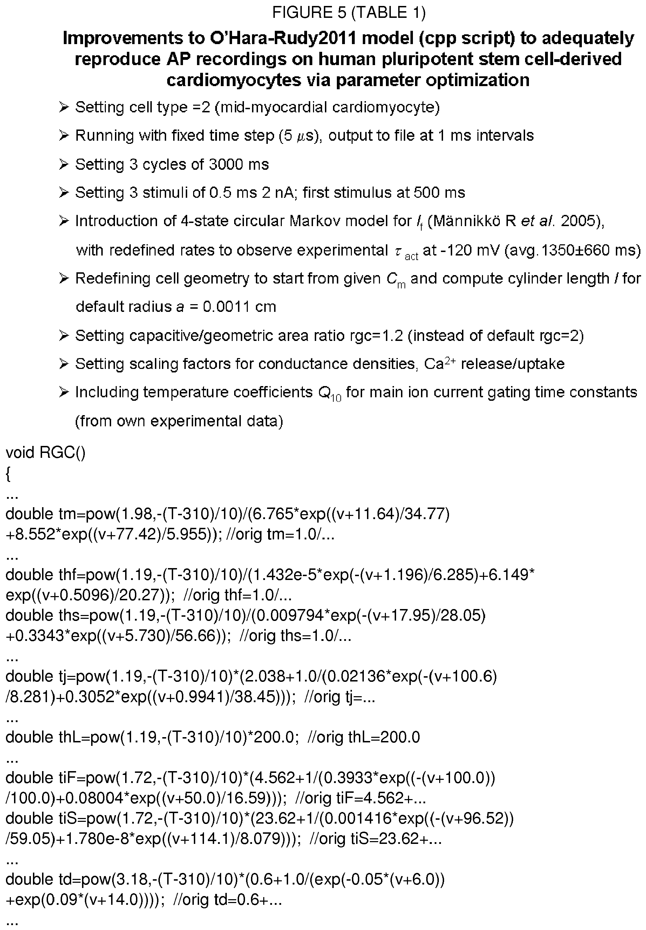

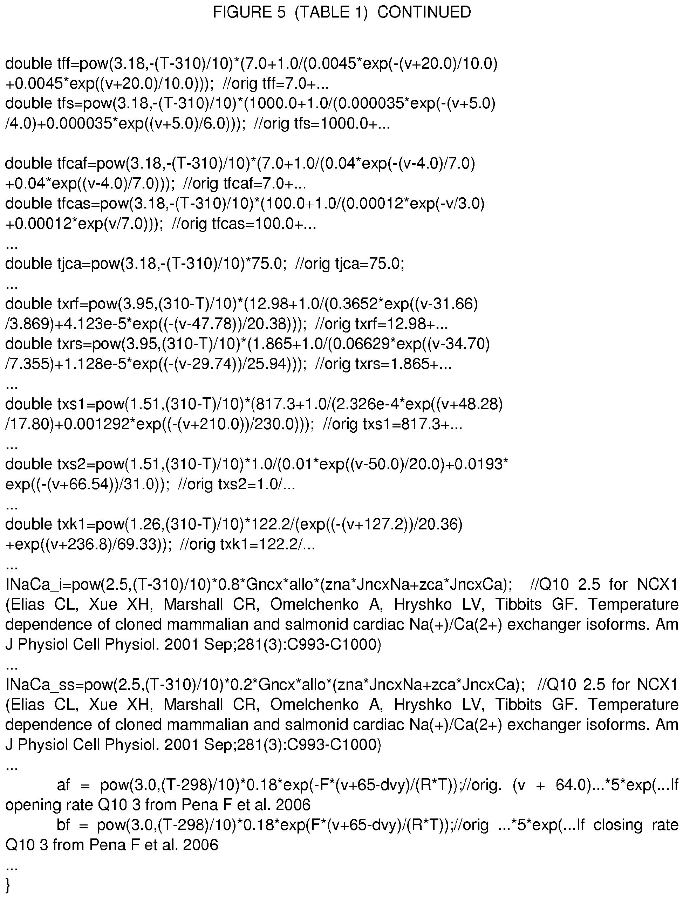

The changes applied to the standard O'Hara-Rudy 2011 model are illustrated in Table 1 (Figure 5): the most important ones are inclusion of a four-state circular allosteric Markov model of $I_f$ gating adapted from (32) with rates adjusted to observe a time constant of $I_f$ activation at - 120 mV of 1350 ms (average in own experiments on human induced pluripotent stem cell-derived cardiomyocytes (hiPSC-CM) at room temperature) and introduction of temperature dependence of gating for most ion current components using $Q_{10}$ values obtained from own experiments or from the literature.

Another change applied to the model is use of fixed internal $Na^+$ and $K^+$ concentrations, equal to concentrations in the internal (pipette) solution, assuming these two cation species diffuse fast enough from pipette to the interior of the cardiomyocyte.

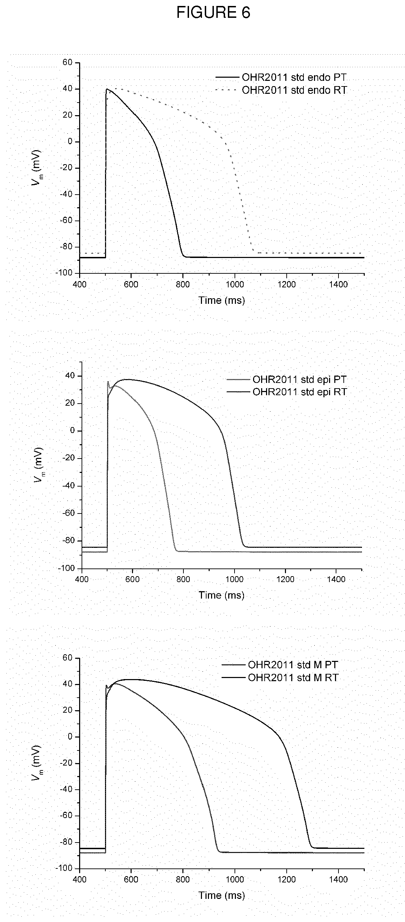

The modified O'Hara-Rudy 2011 model was tested by running numeric integration computations with default stimulus and default parameters for subendocardial, subepicardial and mid-myocardial (M) human ventricular cardiomyocytes, at physiological temperature $PT$ = 37°C = 310 K (default temperature in original O'Hara-Rudy 2011 model) vs. room temperature ($RT$ = 25°C = 298 K), as shown in Figure 6: at $RT$ there is a slow-down of AP time course.

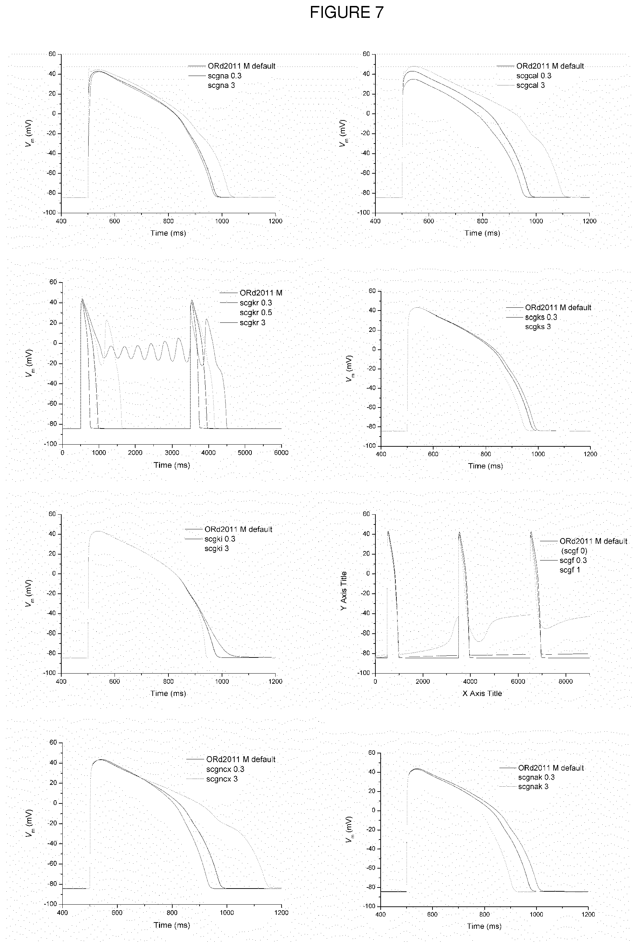

In a set of preliminary tests, the inventors have studied the effects of changing the scaling factors of different ion conductance surface densities on externally paced action potential AP shape and duration; Figure 7 shows effects on AP, computed using default O'Hara-Rudy2011 parameter set for M cell type at $RT$ of change between 0.3 to 3 times the default value of conductance density scaling factors scgna, scgcal, scgkr, scgks, scgki, scgf, scgncx, scgnak.

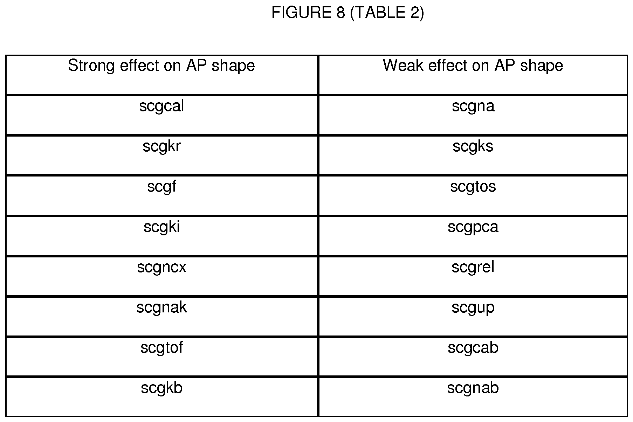

Based of these preliminary tests, in Table 2 (Figure 8) the different scaling factors in the O'Hara-Rudy model are classified into scaling factors with strong effects on externally paced action potential AP shape and duration vs. scaling factors with reduced effects on AP shape and duration. Based on analysis shown in previous figure, ion currents and corresponding ion conductance surface density scaling factors were classified into components with marked effects on the shape and duration of externally paced action potential AP and components with reduced effects. The rapid optimization algorithms were focused only on components with strong effects on externally paced action potential AP shape.

The most important components with marked effects on the shape and duration of externally paced action potential AP shape may be for example scgkr and scgcal, while scgf exerts strong effects on resting potential.

It is a good approximation for parameter optimization algorithms to vary only the scaling factors with marked effects on externally paced action potential (AP) shape.

There are multiple approaches to computational algorithms for fast parameter optimization of the modified O'Hara-Rudy 2011 model to match experimentally recorded externally paced action potential AP shape in a one or several human induced pluripotent stem cell-derived cardiomyocytes hiPSC-CM.

One approach is to run a sequential optimization algorithm, varying the scaling factors one by one at well-defined intervals and in a well-defined order until getting an optimal value for each of them.

The optimality criterion can be the sum of squared errors, i.e. the sum of squared differences between experimentally recorded transmembrane potential and transmembrane potential generated by the modified O'Hara-Rudy model with a given set of parameters, computed at 1-ms time intervals for a total duration of 9 s, using the same standard current stimulus protocol for both the real one or several human induced pluripotent stem cell-derived cardiomyocytes hiPSC-CM and the model (3 current stimuli of 0.5 ms 2 nA amplitude at 3-s intervals, first stimulus at 500 ms).

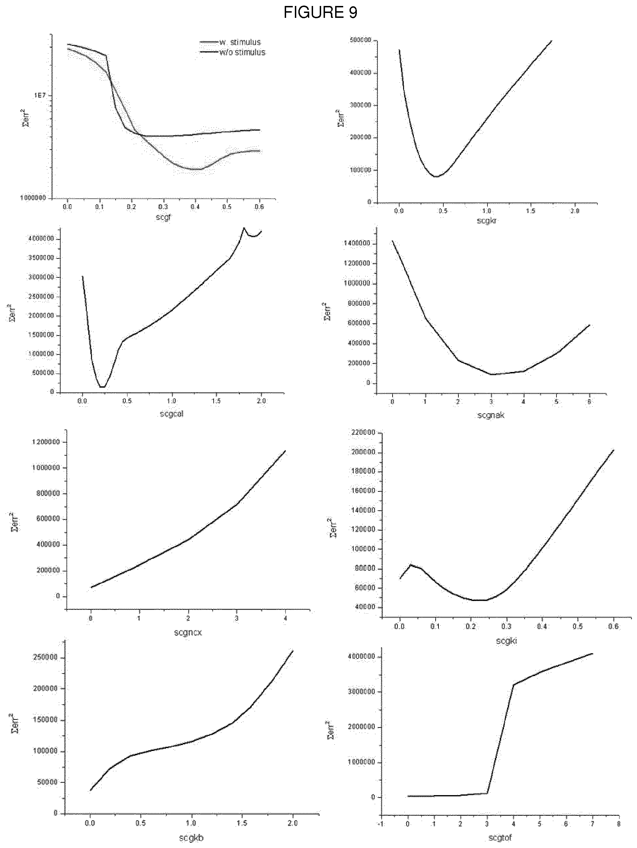

The optimality criterion, such as sum of squared errors for transmembrane potential, landscape for each parameter varied within a certain interval during application of this sequential parameter optimization algorithm for a real experimental dataset is shown in Figure 9: for most parameters a minimum is attained somewhere within the variation interval. The optimality criterion was the sum of squared errors between experimentally-recorded and model-generated transmembrane potential at 1-ms intervals over a 9-s total interval, with the same stimulation protocol applied in the real experiment and to the model (3 current stimuli of 0.5 ms 2 nA at 3-s interval, first stimulus at 500 ms from start). Each parameter was varied in discrete steps over an interval, and generally there was an optimal value where the sum of squared errors reached a minimum for each of them. The order of parameters within the optimization sequence is extremely important, as are the initial estimates of the other parameters beyond the one being optimized.

Another variant of applying the sequential optimization algorithm is in a parallel manner, using parameter order shuffling schemes to obtain different sequences of parameter optimization on different processors (either main processors or graphical processors).

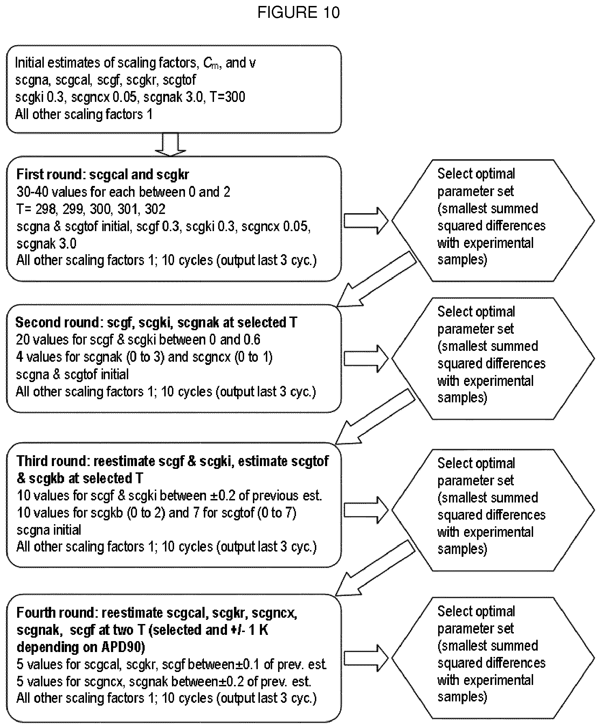

In Figure 10 the general flowchart of a massively parallel variant of the parameter optimization algorithm is shown, comprised of 5 consecutive rounds run on a majority of the 7168 graphical processors of a system equipped with 2 Nvidia® Tesla® P100 GPU accelerator cards. The algorithm includes 5 rounds of optimization, with well-defined combinations of parameters and intervals of variation for each round, based on preliminary results of optimization algorithms with manual sequential change of parameters. After each round the best-fitting set of parameters is used as base for the next round, although some parameters are finely trimmed during late rounds.

Another effective fast optimization method is to use the initial set of scaling factors computed by the automated analysis script of voltage-clamp recordings in a single round, with adequate guesses for the other scaling factors, to inspect visually the computed externally paced action potential AP shape vs. experimental recorded externally paced action potential AP shape, and to perform manually adjustments of some parameters based on knowledge of effects of each parameter on different AP or resting potential features, followed by a second single round of numeric integration; results obtained with this method, while not perfect, provide usually a satisfactory approximation for generating stimulus files for dynamic-clamp protocols.

Another very computationally-effective variant of the optimization algorithm is to use the experimentally recorded series of externally paced action potential AP shape applied directly to the model with parameters estimated by automated analysis of voltage-clamp recordings or reasonably guessed, and to compute the dynamics of each ion current component during this externally given transmembrane potential, using subsequently the output (dynamics of different ion currents) to generate a stimulus file for dynamic-clamp protocols.

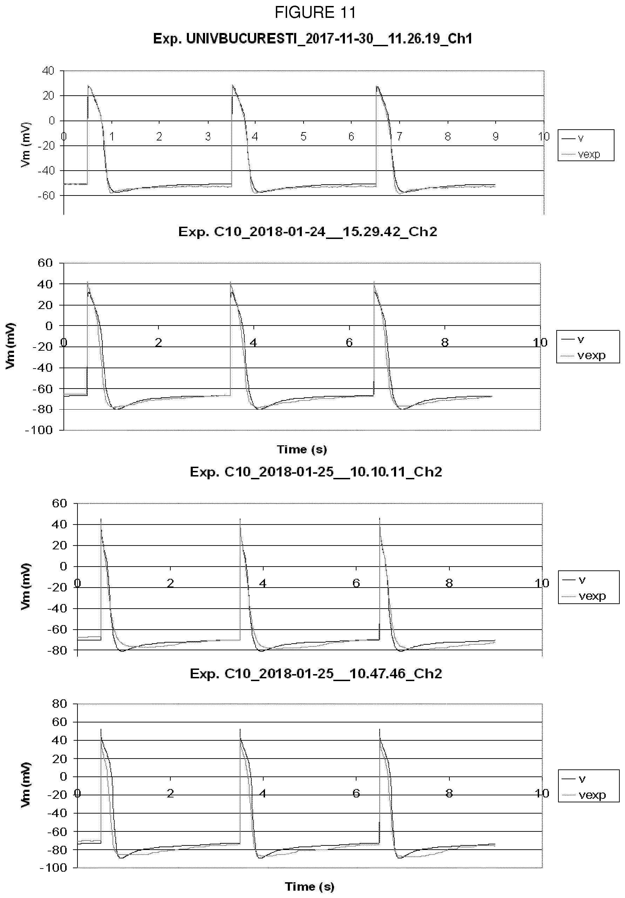

Figure 11 shows the result of applying the sequential monoprocessor parameter optimization algorithm for 4 distinct workings of the method corresponding to real experiments on one or several human induced pluripotent stem cell-derived cardiomyocytes hiPSC-CM: in each case the externally paced action potential AP shape generated by the optimized model fits satisfactorily the experimentally recorded externally paced action potential AP shape. In all 4 distinct experiments, the experimentally recorded externally paced action potential AP was obtained in current-clamp mode using the standard stimulation protocol (3 current stimuli of 0.5 ms 2 nA at 3-s interval, first stimulus at 500 ms from start).

Table 3 (Figure 12) shows the advantages of applying the β-escin-perforated method instead of the classical "ruptured" whole-cell patch-clamp approach in experiments on one or several human induced pluripotent stem cell-derived cardiomyocytes hiPSC-CM: the rates of change (in % change/min. relative to initial values) of peak/steady ion current amplitudes are reduced by almost an order of magnitude for the ion current components exerting the most pronounced effects on AP shape and duration: $I_{CaL}$ and $I_{Kr}$.

A consequence is the improved stability of AP shape and duration in the β-escin-perforated vs. the classical "ruptured" configuration, reflected in the APD90 rate of change (% change/min.) of 0.7%/min. (perforated) vs. 4.6 %/min. ("ruptured") (see Table 3 (Figure 12)), and as shown in Figure 13, illustrating consecutive current-clamp sweeps at 1-min. intervals using the standard stimulus protocol: the gradual AP plateau run-down shown in the upper panel ("ruptured" configuration) is absent in the lower panel (β-escin-perforated configuration).

This major improvement in externally paced action potential AP shape is stability over a 10-20 min. time interval achieved in β-escin-perforated patch-clamp experiments, because only with a stable shape of the externally paced action potential in control conditions proarrhythmogenic effects of the candidate drug on the shape of the externally paced action potential AP can be rigorously characterized and reverted using dynamic-clamp protocols.

After application of the pharmacological compound and distortion of externally paced action potential AP shape by inhibitory effects on one or multiple ion currents, the sequence of three voltage-clamp protocols is reapplied to the patch-clamped one or several human induced pluripotent stem cell-derived cardiomyocytes hiPSC-CM and the inhibitory effects on the five ion currents measured this way can be approximately estimated.

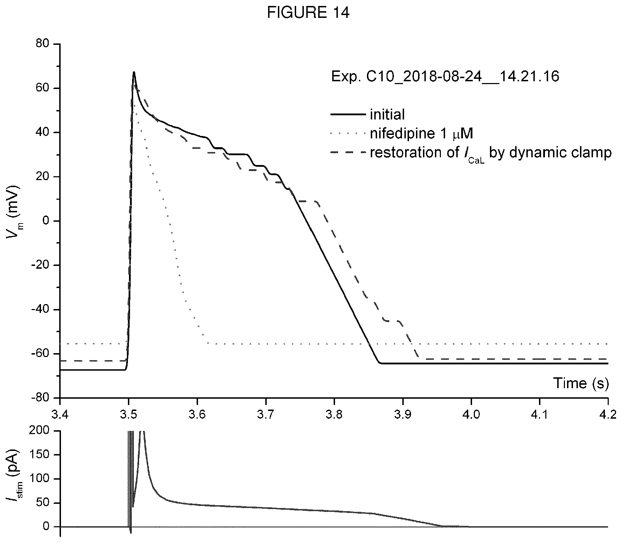

Restoration of the initial externally paced action potential AP shape, as shown in the example illustrated in Figure 14 may represent a validation criterion for the detection of the proarrhythmogenic risk of said drug candidate. The current restauration stimulus contained is shown in the lower panel of Figure 14.

EXAMPLES

The inventors used Cor.4U® cardiomyocytes, a commercial human induced pluripotent stem cell-derived cardiomyocytes hiPSC-CM preparation provided by Ncardia, cultured in multi-well plates precoated with Geltrex® and detached by enzyme treatment with accutase and collagenase II according to manufacturer's instructions.

The experimental phases of the method were performed using a CytoPatch™2 third-generation automated patch-clamp platform based on Cytocentering® technology, using plasma-cleaned standard 2-channel (2K) microfluidic chips with quartz pipette tips of 2.5 µm inner diameter embedded in standard polydimethylsiloxane covers.

The external and internal solutions were physiological and had the following composition (in mM): NaCl 140, KCl 2.5, $CaCl_2$ 2, $MgCl_2$ 2, HEPES 10, glucose 10, pH 7.40 at 25°C titrated with NaOH, osmolality 320 mOsm/kg $H_2O$ adjusted with water / sucrose 1 M (external solution), and $K^+$ gluconate 100, KCl 20, $CaCl_2$ 1, $MgCl_2$ 1, EGTA 11, HEPES 10, $Na_2$ phosphocreatine 3, MgATP 4, pH 7.20 at 25°C titrated with KOH, osmolality 290 mOsm/kg $H_2O$ (internal solution).

The liquid junction potential for this combination of solutions computed with the liquid junction potential calculator application implemented in Clampex 10 (Molecular Devices) (menu: Tools>Junction Potential) was $V_{LJP}$ = 13.4 mV.

The external solution, as well as deionized water used by the CytoPatch™2 equipment were degassed overnight by magnetic stirring in flasks connected to a vacuum pump.

The internal solution was kept at -20°C, thawed prior to beginning of experiments and supplemented with β-escin from an aqueous stock solution to 35 $\mu$M final concentration.

A complex electrophysiology and pharmacology assay combining multiple voltage-clamp, current-clamp and dynamic-clamp protocols predefined in the CytoLogic software controlling the CytoPatch™2 platform was used.

After chip filling and priming, cell catch and gigaseal formation, the standard voltage-clamp protocol for $I_{Na}$ was repeatedly applied to monitor perforation by plotting the timeline of peak $I_{Na}$ amplitude.

When an adequate perforation was achieved, characterized by an access resistance below 20 M$\Omega$, the sequence of voltage-clamp and current-clamp protocols was initiated.

The parameters of the equivalent electrical circuit ($C_m$, $R_a$) as well as the voltage offest ($V_{off}$) were directly read as displayed by the CytoLogic software.

The voltage-clamp recordings performed with the 3 standard voltage protocols illustrated in Figures 2-4, as well as the current-clamp recording using the standard current stimulus protocol (3 current stimuli of 0.5 ms 2 nA amplitude at 3-s intervals, first stimulus at 500 ms) were exported from the standard ccm format of the CytoLogic software to Axon Text File (ATF) format.

Figure 2 depicts the voltage-clamp protocol for measuring peak voltage-dependent Na$^+$ current amplitude (Nav_standard_v2_MC defined in CytoLogic). The upper panel shows the applied voltage and transmembrane current recorded in a Cor.4U® cardiomyocyte by automated patch-clamp. Peak current is measured as negative peak between 140 and 179 ms, base current as average between 174 and 179 ms, whereas the lower panel shows the voltage dependence of activation and steady-state inactivation of cardiac $I_{Na}$ (human Nav1.5) expressed in CHO cells, based on recordings with specific multi-step voltage protocols, fitted with Boltzmann charge-voltage functions.

Figure 3 depicts the voltage-clamp protocol for separating L-type Ca$^{2+}$ current ($I_{CaL}$) from voltage-dependent Na$^+$ current ($I_{Na}$) (CaVNaVSeparation_MC defined in CytoLogic). The upper panel shows the applied voltage and transmembrane current recorded in a Cor.4U® cardiomyocyte by automated patch-clamp with an 11-sweep protocol. After an initial depolarizing pulse (200 ms at -20 mV), during the 100-ms step at -40 mV almost all Na$^+$ channels remain inactivated (see lower panel of Figure 2) while most L-type Ca$^{2+}$ channels get removed from inactivation (see lower panel of this figure), therefore a second depolarizing step activates only $I_{CaL}$ as inward current. Peak current is measured as negative peak between 323-343 ms during the 7th sweep (step at +10 mV) and base current as average between 500-519 ms during the same sweep. The lower panel shows the voltage dependence of activation and steady-state inactivation of $I_{CaL}$ measured with specific voltage protocols and solutions in human induced pluripotent stem cell-derived cardiomyocytes (hiPSC-CM), fitted with Boltzmann charge-voltage functions.

Figure 4 depicts the voltage-clamp protocol for measuring transient outward current ($I_{to}$), delayed rectifier K$^+$ current ($I_{Kr}$+$I_{Ks}$, mainly $I_{Kr}$), and "funny" hyperpolarization-activated current ($I_f$) (hERG_Standard_m120_m50_MC defined in CytoLogic). The upper panel shows the applied voltage and transmembrane current recorded in a Cor.4U® cardiomyocyte by automated patch-clamp with a 2-sweep protocol. $I_{to}$ is measured as positive peak current between 220-500 ms vs. base current measured as average between 10-90 ms during the first sweep, because in the second sweep the outward transient current at +40 mV includes an $I_f$ tail current. $I_{Kr}$+$I_{Ks}$ is measured as peak current at -50 mV during the second sweep, when delayed rectifier K$^+$ channels recover rapidly from inactivation, as positive peak between 2210-3000 ms, vs. base current measured as average between 4000-4199 ms during the same sweep, at the base of exponential deactivation. $I_f$ is measured as steady current level during the first sweep at -120 mV as average between 4100-4199 ms, vs. starting value of exponential activation measured as positive peak between 2210-3000 ms during the same sweep. The lower panel shows the voltage dependence of activation and steady-state inactivation of $I_{Kr}$ based on experiments on HEK293T cells stably expressing hERG1 with specific voltage protocols (left) and voltage dependence of $I_f$ conductance $g_f$ based on experiments in Cor.4U® cardiomyocytes (right), all plots fitted with Boltzmann charge-voltage functions.

The three voltage-clamp recordings in initial conditions were renamed 1.atf, 2.atf, and 3.atf; 3.atf was supplementary processed by low-pass filtering at a cutting frequency of 70 Hz using the Gaussian software filter implemented in Clampfit 10 (Molecular Devices).

The current-clamp recording was also converted to ATF format and supplementary processed by subtracting the offset voltage ($V_{off}$) (menu: Analyze>Adjust baseline>Subtract fixed value), low-pass filtering as described above, and resampled at 1 ms intervals (menu: Analyze>Data reduction>Substitute average>Reduction factor: 10), and then saved after renaming it AP.atf.

The inventors used a C++ script for automated analysis of the three voltage-clamp recordings to compute ion current conductance surface densities and scaling factors relative to default values in the modified O'Hara-Rudy2011 model for the ion currents $I_{Na}$, $I_{CaL}$, $I_{to}$, $I_{Kr}$, and $I_f$, entering manually the $C_m$ and $R_a$ values listed above, read directly from the CytoLogic Measured Data window.

The output of the analysis script was written in the file out.dat and used as input for the parameter optimization algorithm.

The inventors then applied a simplified version of the optimization algorithm, running initially a single numeric integration with fixed time step (5 $\mu$s) over a total 9-s time interval, using an identical current stimulus protocol as in the real experiment (3 current stimuli of 0.5 ms 2 nA amplitude at 3-s intervals, first stimulus at 500 ms), and using the five ion conductance surface density scaling factors provided by the automated analysis script, the other scaling factors being reasonably guessed.

The output file generated by the optimization algorithm after this first round (a file named output.txt containing time intervals, voltage from experiment - file AP.atf and generated by the model, as well as values of different ion currents, calcium fluxes, ion concentrations, etc.) was imported in Excel, and the experimental and model-generated transmembrane potential over 9 s were plotted in parallel.

Visual inspection of these time plots was used to adjust some parameters, knowing in advance the effects of each parameter on different features of AP and resting potential.

The model with manually readjusted parameters was run once more, and the newly generated file output.txt was reopened in Excel to assess the results.

During the 10-min. time interval required to perform this automated analysis and parameter optimization, the Cor.4U® cardiomyocyte was challenged with nifedipine 1 $\mu$M, with repeated current-clamp recordings at 1-min. intervals to monitor the effect of nifedipine on AP shape.

The file output.txt in its final version generated by the optimized model was used with another automated C++ script to generate a current stimulus file (stimfile.csv) in a standard format readable by the CytoLogic software.

This restauration stimulus file contains the sum of ion currents inhibited by the candidate drug (based on percentages of inhibition for each current component defined directly in the script - in this case 100% for $I_{CaL}$ only) with reversed sign and completed by the three standard current stimuli (0.5 ms 2 nA amplitude at 3-s intervals, first stimulus at 500 ms).

The stimulus file was loaded in CytoLogic and applied to the patch-clamped cardiomyocyte in a dynamic-clamp protocol, in order to restore the initial externally paced action potential (AP) shape in the one or several human induced pluripotent stem cell-derived cardiomyocytes hiPSC-CM subjected to the action of the drug candidate.

Results of the examples

The parameters of the equivalent electrical circuit in one experiment (C10_2018-08-24_14.21.16_Ch1) were as follows: $C_m$ = 65.62 pF, $R_a$ = 12.05 M$\Omega$, $V_{off}$ = 44.18 mV.

The computation of each ion current conductance surface density and scaling factor by the C++ automated analysis script occurred as follows.

For $I_{Na}$: voltage-clamp recording using standard CytoLogic protocol Nav_standard_v2_MC (file 1.atf) was analyzed as shown in Figure 2:

- peak Na$^+$ current was measured as negative peak current over the time interval between 140-179 ms: peak_current_early = -6699.82 pA
- base current was measured at the base of exponential inactivation during the -10 mV step as average over the time interval 174-179 ms: late_current = -30.52 pA
- peak_current_corr = peak_current_early - late_current = -6669.3 pA
- corrected voltage of depolarizing step: $V_m$ (peak) = -10 - (-6.69982 nA x 12.05 M$\Omega$) - 13.4 mV = 57.33 mV
- corrected holding voltage: $V_m$ (base) = -90 - (-0.03052 nA x 12.05 M$\Omega$) - 13.4 mV = - 103.03 mV
- activation and steady-state (removal from) inactivation probabilities computed with Boltzmann charge-voltage functions shown in lower panel of Figure 2:

$$P_a = 1/(1 + \exp(-(V_m \text{ (peak)} + 38.5)/6.01998) = 1$$

$$P_{ssi} = 1/(1 + \exp((V_m \text{ (base)} + 75.4)/5.1242) = 0.9956$$

- reversal potential for Na$^+$ in given conditions: $E_{Na} = RT/F \ln ([Na^+]_o/[Na^+]_i) = 81.85$ mV
- driving force: $DF = V_m$ (peak) - $E_{Na}$ = 57.33 mV - 81.85 mV = -24.52 mV
- conductance surface density: $g_{Na}$ = peak_current_early_corr (pA) $/DF/C_m/P_A/P_{ssi}$ = 4.163 nS/pF (equivalent to mS/$\mu$F)
- scaling factor (relative to default value in O'Hara-Rudy2011 model for M type: $g_{Na}$ = 75 mS/$\mu$F): scgna = 0.0555

For $I_{CaL}$: voltage-clamp recording using standard CytoLogic protocol CaVNaVSeparation_MC (file 2.atf) (7th sweep at +10 mV) was analyzed as shown in Figure 3:

- peak $Ca^{2+}$ current was measured as negative peak current over the time interval between 323-343 ms: peak_current = -113.73 pA
- base current was measured at the base of exponential inactivation during the +10 mV step as average over the time interval 500-519 ms: base_current = 9.64 pA
- peak_current_corr = peak_current - base_current = -104.09 pA
- corrected voltage of depolarizing step: $V_m$ (peak) = 10 - (-0.11373 nA x 12.05 MΩ) - 13.4 mV = -2.03 mV
- corrected holding voltage: $V_m$ (base) = -40 - 13.4 mV = -53.4 mV
- activation and steady-state (removal from) inactivation probabilities computed with Boltzmann charge-voltage functions shown in lower panel of Figure 3:

$$P_a = 1/(1 + \exp(-(V_m \text{ (peak)} + 15.2)/5.2) = 0.9264$$

$$P_{ssi} = 1/(1 + \exp((V_m \text{ (base)} + 31.1)/7.6) = 0.9495$$

- corrected peak $I_{CaL}$ surface density:
  ilcapeak = peak_current_corr (pA)/$C_m$/$P_A$/$P_{ssi}$ = -1.803 pA/pF
- scaling factor for $I_{CaL}$ (by dividing to peak $I_{CaL}$ surface density value for an activating step to - 4.418 mV in the O'Hara-Rudy2011 model for mid-myocardial cardiomyocytes ilcapeak = - 7.07063 pA/pF): scgcal = 0.255

For $I_{to}$: voltage-clamp recording using standard CytoLogic protocol hERG_Standard_m120_m50_MC (file 3.atf) (first sweep) was analyzed as shown in Figure 4:

- peak $I_{to}$ was measured as positive peak current over the time interval between 220-500 ms: Ito_peak = 0 pA (no peak $I_{to}$ was detected in this experiment)
- base current was measured as average over the time interval 10-90 ms during the 100-ms step at -70 mV: Ito_base = -26.99 pA
- Ito_peak_corr = Ito_peak - Ito_base (in this case no Ito_peak was detected, therefore Ito_peak_corr = 0 pA)

  - corrected voltage of depolarizing step: $V_m$ (peak) = 40 - (0 nA x 12.05 MΩ) - 13.4 mV = 26.6 mV
  - corrected holding voltage: $V_m$ (base) = -70 - 13.4 mV = -83.4 mV

- activation and steady-state (removal from) inactivation probabilities computed with Boltzmann charge-voltage functions from (34):

$$P_a = 1/(1 + \exp(-(V_m \text{ (peak)} + 3)/15) = 0.88$$

$$P_{ssi} = 1/(1 + \exp((V_m \text{ (base)} + 33.5)/10) = 0.99$$

  - reversal potential for $K^+$ in given conditions: $E_K = RT/F \ln ([K^+]_o/[K^+]_i) = -99.49$ mV

- driving force: $DF = V_m$ (peak) - $E_K$ = 26.6 mV - (-99.49 mV) = 126.09 mV
- conductance surface density: $g_{to}$ = Ito_peak_corr (pA) /$DF$/$C_m$/$P_a$/$P_{ssi}$ = 0 nS/pF
- scaling factor (relative to default value in O'Hara-Rudy2011 model for M type: $g_{to}$ = 0.08 mS/μF): scgtof = 0

For $I_{Kr}$: voltage-clamp recording using standard CytoLogic protocol hERG_Standard_m120_m50_MC (file 3.atf) (second sweep) was analyzed as shown in Figure 4:

- peak $I_{Kr}$ was measured as positive peak current over the time interval between 2210-3000 ms (assuming the delayed rectifier peak current at the beginning of the voltage step to -50 mV is contributed entirely by $I_{Kr}$): IKrpeak = 15.78 pA
- base current was measured as average over the time interval 4000-4199 ms at the base of the exponential deactivation during the 2-s step at -50 mV: IKrbase = 2.29 pA
- IKrcorr = IKrpeak - IKrbase = 13.49 pA
- corrected voltage of repolarizing step: $V_m$ (peak) = -50 - (0.01349 nA x 12.05 MΩ) - 13.4 mV =
- 63.6 mV

- corrected holding voltage: $V_m$ (base) = 40 - 13.4 mV = 26.6 mV

- activation and steady-state (removal from) inactivation probabilities computed with Boltzmann charge-voltage functions shown in lower panel of Figure 3:

$$P_a = 1/(1 + \exp(-(V_m \text{ (base)} + 40.3)/9.18) = 0.999$$

$$P_{ssi} = 1/(1 + \exp((V_m \text{ (peak)} + 25.2)/23.8) = 0.83$$

- driving force: $DF = V_m - E_K$ = -63.5 mV - (-99.49 mV) = 35.99 mV
- conductance surface density: $g_{Kr}$ = IKrcorr (pA) $/DF/C_m/P_A/P_{ssi}$ = 0.00689 nS/pF
- scaling factor (relative to default value in O'Hara-Rudy2011 model for M type: $g_{Kr}$ = 0.0368 mS/$\mu$F): scgkr = 0.187

For $I_f$: voltage-clamp recording using standard CytoLogic protocol hERG_Standard_m120_m50_MC (file 3.atf) (first sweep) was analyzed as shown in Figure 4:

- steady $I_f$ was measured as average current over the time interval between 4100-4199 ms: Ifstdy = -118.93 pA
- the starting point of the exponential $I_f$ activation Ifstart was considered the positive peak value over the time interval between 2210-3000 ms: Ifstart = -56.10 pA
- Ifcorr = Ifstdy - Ifstart = -62.83 pA
- corrected voltage of the hyperpolarizing step:
  $V_m$ (steady) = -120 - 13.4 mV = -133.4 mV
- steady-state $I_f$ activation probability $P_a$ at the corrected voltage step was computed using a Boltzmann charge-voltage function similar to that obtained by fitting the $I_f$ conductance-voltage plot shown in lower panel of Figure 3:

$$P_a = 1/(1 + \exp((V_m \text{ (steady)} + 96.2)/10.3) = 0.97$$

- reversal potential of $I_f$ computed with a Goldmann-Hodgkin-Katz voltage equation: $E_m = RT/F$

In $((P_{Na}[Na^+]_o + P_K[K^+]_o)/(P_{Na}[Na^+]_i + P_K[K^+]_i))$= -7.44 mV
taking permeabilities proportional to $I_f$ relative conductances $g_{h,Na}$=0.3833$g_h$ and $g_{h,K}$=0.6167$g_h$ from (31)

- driving force:

$$DF = V_m \text{ (steady)} - E_m = -133.4 \text{ mV} - (-7.44 \text{ mV}) = -125.96 \text{ mV}$$

- conductance surface density: $g_f$ = Ifcorr (pA) $/DF/C_m/P_A$ = 0.00784 nS/pF
- scaling factor (relative to default value implemented in modified O'Hara-Rudy2011 model: $g_f$ = 0.06 mS/$\mu$F): scgf = 0.131

The scaling factors for these five ion current components computed automatically by the analysis algorithm were extracted from the output file out.dat and inserted in the C++ script of the modified O'Hara-Rudy2011 model used for parameter optimization; the other scaling factors were set to convenient guessed values.

For parameter optimization a rapid two-step approach was used: in the first round estimated parameters were used, yielded by the automated analysis script, and in the second round some parameters were manually adjusted after inspecting the plots of experimentally recorded and model-generated transmembrane potential over 9s applying the same current stimulus protocol.

The output.txt file generated in the last optimization round was used with another automated C++ script to compute the stimulus file stimfile.csv, including 100% of the $I_{CaL}$ component with reversed sign completed with the three brief current stimuli (0.5 ms 2 nA) present in the standard current-clamp protocol.

The current restauration stimulus contained in stimfile.csv is shown in the lower panel of Figure 14.

The initial shape of the AP is shown in black continuous line in Figure 14.

While the optimization was performed the Cor.4U® cardiomyocyte was challenged with nifedipine 1 $\mu$M, which produced complete block of $I_{CaL}$ and disappearance of the AP plateau (dotted trace in Figure 14)

By applying the stimulus current waveform from the stimulus file stimfile.csv in a dynamic-clamp protocol restoration of initial externally paced action potential AP shape was obtained (dashed line in Figure 14).

References

1. S. Polak, M. K. Pugsley, N. Stockbridge, C. Garnett, and B. Wiśniowska, AAPS J, 2015, 17, 1025-32.
2. B. Amuzescu, O. Scheel, and T. Knott, J Phys Chem Biophys., 2014, 4, 1-4.
3. D. Noble, J Pharmacol Sci., 2008, 107, 107-17.
4. J. Kramer, C. A. Obejero-Paz, G. Myatt, Y. A. Kuryshev, A. Bruening-Wright, J. S. Verducci, and A. M. Brown, Sci Rep, 2013, 3.
5. P. T. Sager, G. Gintant, J. R. Turner, S. Pettit, and N. Stockbridge, Am Heart J, 2014, 167, 292-300.
6. T. O'Hara, L. Virag, A. Varró, and Y. Rudy, PLoS Comput Biol, 2011, 7, 26.
7. R. Wallis, C. Benson, B. Darpo, G. Gintant, Y. Kanda, K. Prasad, D. G. Strauss, and J. P. Valentin, J Pharmacol Toxicol Methods, 2018, 8719, 30626-9.
8. H. Huang, M. K. Pugsley, B. Fermini, M. J. Curtis, J. Koerner, M. Accardi, and S. Authier, J Pharmacol Toxicol Methods, 2017, 87, 11-23.
9. M. J. Windley, N. Abi-Gerges, B. Fermini, J. C. Hancox, J. I. Vandenberg, and A. P. Hill, J Pharmacol Toxicol Methods, 2017, 87, 99-107.
10. Z. Li, S. Dutta, J. Sheng, P. N. Tran, W. Wu, K. Chang, T. Mdluli, D. G. Strauss, and T. Colatsky, Circ Arrhythm Electrophysiol, 2017, 10, 004628.
11. Z. Li, S. Dutta, J. Sheng, P. N. Tran, W. Wu, and T. Colatsky, J Pharmacol Toxicol Methods, 2016, 81, 233-9.
12. S. Dutta, K. C. Chang, K. A. Beattie, J. Sheng, P. N. Tran, W. W. Wu, M. Wu, D. G. Strauss, T. Colatsky, and Z. Li, Front, 2017, 8:616., 10.3389/fphys.2017.00616. eCollection 2017.
13. K. H. Gilchrist, G. F. Lewis, E. A. Gay, K. L. Sellgren, and S. Grego, Toxicol Appl Pharmacol, 2015, 288, 249-57.
14. K. Harris, M. Aylott, Y. Cui, J. B. Louttit, N. C. McMahon, and A. Sridhar, Toxicol Sci, 2013, 134, 412-26.
15. A. Mehta, Y. Chung, G. L. Sequiera, P. Wong, R. Liew, and W. Shim, Toxicol Sci, 2013, 131, 458-69.
16. P. Mulder, T. de Korte, E. Dragicevic, U. Kraushaar, R. Printemps, M. L. H. Vlaming, S. R. Braam, and J. P. Valentin, J Pharmacol Toxicol Methods, 2018, 91, 36-42.
17. C. T. Bot, K. Juhasz, F. Haeusermann, L. Polonchuk, M. Traebert, and S. Stoelzle-Feix, J Pharmacol Toxicol Methods, 2018, 8719, 30600-2.
18. H. M. Himmel, J Pharmacol Toxicol Methods, 2013, 68, 97-111.
19. N. Hu, T. Wang, H. Wan, L. Zhuang, R. Kettenhofen, X. Zhang, Y. S. Zhang, W. Xu, M. Gossmann, H. Bohlen, X. Hou, and P. Wang, Biosens Bioelectron, 2018, 117, 354-365.
20. B. Koci, G. Luerman, A. Duenbostell, R. Kettenhofen, H. Bohlen, L. Coyle, B. Knight, W. Ku, W. Volberg, J. R. Woska, Jr., and M. P. Brown, Toxicol Appl Pharmacol, 2017, 329, 121-127.
21. X. Zhang, L. Guo, H. Zeng, S. L. White, M. Furniss, B. Balasubramanian, E. Lis, A. Lagrutta, F. Sannajust, L. L. Zhao, B. Xi, X. Wang, M. Davis, and Y. A. Abassi, J Pharmacol Toxicol Methods, 2016, 81, 201-16.
22. S. Bedut, C. Seminatore-Nole, V. Lamamy, S. Caignard, J. A. Boutin, O. Nosjean, J. P. Stephan, and F. Coge, Am J Physiol Heart Circ Physiol, 2016, 311, 3.
23. K. Blinova, J. Stohlman, J. Vicente, D. Chan, L. Johannesen, M. P. Hortigon-Vinagre, V. Zamora, G. Smith, W. J. Crumb, L. Pang, B. Lyn-Cook, J. Ross, M. Brock, S. Chvatal, D. Millard, L. Galeotti, N. Stockbridge, and D. G. Strauss, Toxicol Sci, 2017, 155, 234-247.
24. P. W. Burridge, S. Thompson, M. A. Millrod, S. Weinberg, X. Yuan, A. Peters, V. Mahairaki, V. E. Koliatsos, L. Tung, and E. T. Zambidis, PLoS One, 2011, 6, 0018293.
25. M. P. Hortigon-Vinagre, V. Zamora, F. L. Burton, J. Green, G. A. Gintant, and G. L. Smith, Toxicol Sci, 2016, 154, 320-331.
26. A. Lopez-Izquierdo, M. Warren, M. Riedel, S. Cho, S. Lai, R. L. Lux, K. W. Spitzer, I. J. Benjamin, M. Tristani-Firouzi, and C. J. Jou, Am J Physiol Heart Circ Physiol, 2014, 307, 29.
27. E. R. Pfeiffer, R. Vega, P. M. McDonough, J. H. Price, and R. Whittaker, J Pharmacol Toxicol Methods, 2016, 81, 263-73.
28. H. Zeng, M. I. Roman, E. Lis, A. Lagrutta, and F. Sannajust, J Pharmacol Toxicol Methods, 2016, 81, 217-22.
29. W. R. Giles and D. Noble, Biophys J, 2016, 110, 278-80.
30. US2013153067(A1)
31. Y. Kurata, I. Hisatome, S. Imanishi, and T. Shibamoto, Am J Physiol Heart Circ Physiol., 2002, 283, H2074-101. doi: 10.1152/ajpheart.00900.2001
32. O. Scheel, S. Frech, B. Amuzescu, J. Eisfeld, K. H. Lin, and T. Knott, Assay Drug Dev Technol, 2014, 12, 457-69.

33. R. Männikkö, S. Pandey, H. P. Larsson, and F. Elinder, J Gen Physiol., 2005, 125, 305-26. doi: 10.1085/jgp.200409130. Epub 2005 Feb 14.

34. T. R. Shannon, F. Wang, J. Puglisi, C. Weber, and D. M. Bers, Biophys J., 2004, 87, 3351-71. doi: 10.1529/biophysj.104.047449. Epub 2004 Sep 3.

**Claims**

1. Method of detecting *in vitro* the proarrhythmogenic risk of a drug candidate *wherein* by use of a high-performance automated patch-clamp platform (APCP) able to carry out experimental measurements and recordings of at least five human ventricular ion currents and of an externally paced action potential (AP) on human induced pluripotent stem cell-derived cardiomyocytes (hiPSC-CM) and by use of a computerized algorithm running on a high-performance computer unit (HPCU) for rapid parameter optimization of an advanced cardiomyocyte electrophysiology mathematical model to accurately reproduce the shape of said externally paced action potential (AP), the following one-shot sequence of steps is carried out on said human induced pluripotent stem cell-derived cardiomyocytes (hiPSC-CM):

   Step1- Capturing on a microfluidic chip of one or several human induced pluripotent stem cell-derived cardiomyocytes (hiPSC-CM) from a suspension, forming of gigaseals,
   followed by achieving of whole-cell configuration by β-escin perforation, which allows to stabilize the externally paced action potential (AP) shape over a duration generally between 10 and 20 minutes;
   Step 2- Separating, recording, and measuring peak or steady-state amplitudes of the at least five ion currents by applying to said one or several human induced pluripotent stem cell-derived cardiomyocytes (hiPSC-CM) approached in β-escin perforated whole-cell patch-clamp of a series of well-defined voltage-clamp protocols.
   Step 3- Computing based on automated analysis of recordings using the series of said voltage-clamp protocols of ion conductance surface densities for each of the at least five said ion currents and of scaling factors for ion conductance surface densities of each of said at least five ion currents relative to default values of said advanced cardiomyocyte electrophysiology mathematical model.
   Step 4 - Measuring and recording an initial externally paced action potential (AP) of said one or several human induced pluripotent stem cell-derived cardiomyocytes (hiPSC-CM) by applying a standard current-clamp protocol with a single sweep and with predetermined current stimuli.
   Step 5

      - 5a - Applying said drug candidate at a given concentration on one or several human induced pluripotent stem cell-derived cardiomyocytes (hiPSC-CM) fulfilling 2 conditions, namely having a resting potential of < -50mV and a having a ventricular-like initial externally paced action potential (AP) with an Action Potential Duration ratio APD50/APD90 >70% , said application of the drug candidate being for a pre-determined period of time, preferably 8 to 15 minutes, and more preferably 10 minutes, and simultaneously, during the same pre-determined period of time:
      - 5b- Measuring and recording the modified externally paced action potential (AP) under the effect of said drug candidate by applying the same current-clamp protocol as for determining initial externally paced action potential (AP), periodically at fixed intervals of time, preferably one-minute, and detecting proarrhythmogenic effects of said candidate drug on the initial externally paced action potential (AP) shape, such as early or delayed afterdepolarizations;
      - 5c- Rapid optimization of parameters of said advanced cardiomyocyte electrophysiology mathematical model using the said scaling factors for ion conductance surface densities of each of said at least five ion currents in order to accurately reproduce the shape of said externally paced initial action potential (AP) on human induced pluripotent stem cell-derived cardiomyocytes (hiPSC-CM) having as result a model-generated shape of said externally paced initial action potential (AP);

      Step 6 - Measuring and recording amplitudes of the at least five ion currents inhibited after application of said drug candidate using the same series of voltage-clamp protocols as in step 2, comparing them with initial amplitudes in the absence of said drug candidate and calculating percentages of inhibition for each of them;
      Step 7 - Generating a restauration current stimulus file representing the sum of all ion currents inhibited by the candidate drug with reversed sign and the initial predetermined stimuli, to be used further to restore the initial externally paced action potential (AP) shape in the one or several human induced pluripotent stem cell-derived cardiomyocytes (hiPSC-CM) subjected to the action of the drug candidate by running a simulation with said advanced cardiomyocyte electrophysiology mathematical model with previously optimized parameters and the percentages of inhibition calculated in the previous step or known in advance from previous experiments;

Step 8 - Restoring the modified externally paced action potential (AP) to its initial shape, recorded before the application of said drug candidate by applying of the restauration current stimulus file using a dynamic-clamp protocol to the same one human induced pluripotent stem cell-derived cardiomyocyte (hiPSC-CM) on which the drug candidate was applied;

Step 9 Using the calculated percentages of inhibition to compute metrics predictive of proarrhythmogenic risk of said drug candidate such as the net charge integrated over an action potential carried by the sum of several inward and outward ion currents.

2. Method according to claim 1 *wherein* $\beta$-escin perforated whole-cell patch-clamp on the one or several human induced pluripotent stem cell-derived cardiomyocytes (hiPSC-CM) is used in order to stabilize the initial externally paced action potential (AP) shape and the initial peak or steady-state amplitudes of said at least five ion currents over a time interval of 10-20 min. in control conditions, preventing the run-down phenomenon accompanied by progressive shortening of said initial externally paced action potential (AP) shape that occurs in the classical "ruptured" whole-cell patch-clamp approach on said one or several human induced pluripotent stem cell-derived cardiomyocytes (hiPSC-CM).

3. Method according to claim 1 and 2 *wherein* computation of parameters including ion conductance surface densities and scaling factors relative to the advanced cardiomyocyte electrophysiology mathematical model, such as preferably modified O'Hara-Rudy2011 model, of at least five human ventricular ion currents may be achieved in the series of voltage-clamp protocols by using physiological external, respectively extracellular, and internal, respectively pipette solutions, and in the absence of specific blockers, upon measurement of peak or steady-state amplitudes of said at least five ion currents, including at least:

   - peak rapid voltage-dependent inward Na$^+$ current ($I_{Na}$) with scaling factor of conductance density scgna
   - peak L-type inward Ca$^{2+}$ current ($I_{CaL}$) with scaling factor of conductance density scgcal
   - transient outward K$^+$ current ($I_{to}$) with scaling factor of conductance density scgto
   - "funny" hyperpolarization-activated current ($I_f$) with scaling factor of conductance density scgf
   - a sum of the rapid ($I_{Kr}$) and slow ($I_{Ks}$) components of the delayed rectifier K$^+$ current, but where usually $I_{Kr}$ is the dominant component in control conditions, with scaling factor of conductance density scgkr.

4. Method according to any preceding claims, *wherein* the optimization of one or several parameters of said advanced cardiomyocyte electrophysiology mathematical model to accurately reproduce the shape of said initial action potential (AP), such as for example scaling factors of conductance surface densities and temperature, is performed in a sequential, parallel, or combined manner on the high-performance computer unit (HPCU), taking as optimality criterion the sum of squared differences between the experimentally recorded initial externally paced action potential (AP) and the model-generated shape of said externally paced initial action potential (AP) and using some or all the previously computed scaling factors.

5. Method according to any preceding claims, *wherein* the restauration current stimulus file, representing the sum of all ion currents inhibited by said drug candidate, as generated by said advanced cardiomyocyte electrophysiology mathematical model may be used as a validation criterion for the detection of the proarrhythmogenic risk of said drug candidate.

**Patentansprüche**

1. Verfahren zur *in vitro* Detektion des proarrhythmogenen Risikos eines Arzneimittelkandidaten, wobei unter Verwendung einer automatisierten Hochleistungs-Patch-Clamp-Plattform (APCP), die in der Lage ist, experimentelle Messungen und Aufzeichnungen von mindestens fünf menschlichen ventrikulären Ionenströmen, und eines von außen stimulierten Aktionspotentials (AP) an humanen, von induzierten pluripotenten Stammzellen abgeleiteten Kardiomyozyten (hiPSC-CM) durchzuführen, und unter Verwendung eines computergestützten Algorithmus, der auf einer Hochleistungscomputereinheit (HPCU) zur schnellen Parameteroptimierung eines fortgeschrittenen mathematischen Modells für die Kardiomyozyten-Elektrophysiologie ausgeführt wird, um die Form des extern stimulierten Aktionspotentials (AP) exakt zu reproduzieren, die folgende einmalige Abfolge von Schritten an den humanen, von induzierten pluripotenten Stammzellen abgeleiteten Kardiomyozyten (hiPSC-CM) ausgeführt wird:

   Schritt 1 - Einfangen von einem oder mehreren humanen, von induzierten pluripotenten Stammzellen abgeleiteten Kardiomyozyten (hiPSC-CM) aus einer Suspension auf einem Mikrofluidik-Chip, Bilden von Gigaseals, gefolgt vom Erreichen einer Ganzzell-Konfiguration durch β-Escin-Perforation, mit der es möglich ist, die Form des extern stimulierten Aktionspotentials (AP) über eine Dauer von in der Regel zwischen 10 und 20 Minuten zu stabilisieren;

Schritt 2 - Trennen, Aufzeichnen und Messen der Spitzen- oder stationären Amplituden der mindestens fünf Ionenströme, indem auf eine oder mehrere hiPSC-CM im β-Escin perforierten Ganzzell Patch-Clamp eine Reihe von wohldefinierten Spannungs-Klemme-Protokollen angelegt werden;

Schritt 3 - auf einer automatisierten Analyse basierendes Berechnen von Aufzeichnungen unter Verwendung der Serie der Spannungs-Klemme-Protokolle von Ionenleitfähigkeits-Oberflächendichten für jeden der mindestens fünf Ionenströme und Skalierungsfaktoren für die Ionenleitfähigkeits-Oberflächendichten von jedem der mindestens fünf Ionenströme relativ zu Standardwerten des fortgeschrittenen mathematischen Modells der Kardiomyozyten-Elektrophysiologie;

Schritt 4 - Messen und Aufzeichnen eines anfänglichen extern stimulierten Aktionspotentials (AP) der einen oder mehreren humanen, von induzierten pluripotenten Stammzellen abgeleiteten Kardiomyozyten (hiPSC-CM) durch Anwenden eines standardisierten Strom-Klemme-Protokolls mit einem einzigen Durchlauf und mit vorbestimmten Strom-Stimuli;

Schritt 5

- 5a - Applikation des Arzneimittelkandidaten in einer vorgegebenen Konzentration auf einen oder mehrere humane, von induzierte pluripotente Stammzellen abgeleitete Kardiomyozyten (hiPSC-CM), die zwei Bedingungen erfüllen, nämlich erstens ein Ruhepotential von < -50 mV aufweisen und zweitens ein ventrikulär ähnliches, anfängliches extern stimuliertes Aktionspotential (AP) mit einem Verhältnis der Aktionspotentialdauern (Action Potential Duration) APD50/APD90 >70% aufweisen, wobei die Applikation des Arzneimittelkandidaten für einen vorbestimmten Zeitraum, bevorzugt 8 bis 15 Minuten, idealerweise 10 Minuten, und simultan während der gleichen vorbestimmten Zeitdauer erfolgt:

- 5b - Messen und Aufzeichnen des modifizierten extern stimulierten Aktionspotentials (AP) unter der Einwirkung des Arzneimittelkandidaten durch Anwenden des gleichen Strom-Klemme-Protokolls wie zum Bestimmen des anfänglichen extern stimulierten Aktionspotentials (AP), periodisch in festgelegten Zeitintervallen, bevorzugt eine Minute, und Detektieren proarrhythmogener Wirkungen des Kandidaten-Arzneimittels auf die Form des anfänglichen, extern stimulierten Aktionspotentials (AP), wie beispielsweise frühe oder verzögerte Nachdepolarisationen;A

- 5c - Schnelle Optimierung von Parametern des fortschrittlichen mathematischen Modells der Kardiomyozyten-Elektrophysiologie unter Verwendung der Skalierungsfaktoren für Ionenleitfähigkeits-Oberflächendichten jedes der mindestens fünf Ionenströme, um die Form des extern stimulierten anfänglichen Aktionspotentials (AP) an humanen, von induzierten pluripotenten Stammzellen abgeleiteten Kardiomyozyten (hiPSC-CM), die als Ergebnis eine modellgenerierte Form des extern stimulierten anfänglichen Aktionspotentials (AP) aufweisen, exakt zu reproduzieren;

Schritt 6 - Messen und Aufzeichnen von Amplituden der mindestens fünf Ionenströme, die nach Applikation des Arzneimittelkandidaten unter Verwendung der gleichen Reihe von Spannungs-Klemme-Protokollen wie in Schritt 2 gehemmt wurden, durch Vergleichen von diesen mit anfänglichen Amplituden in Abwesenheit des Arzneimittelkandidaten und
Berechnen prozentualer Anteile der Hemmung für jeden von ihnen;

Schritt 7 - Erzeugen einer Datei des Restaurations-Strom-Stimulus, welche die Summe aller Ionenströme, die durch das Kandidaten-Arzneimittel mit umgekehrtem Vorzeichen sowie die zu Anfang vorbestimmten Stimuli darstellt, um weiter verwendet zu werden, um die Form des anfänglichen extern stimulierten Aktionspotentials (AP) in den einen oder mehreren humanen, von induzierte pluripotenten Stammzellen abgeleiteten Kardiomyozyten (hiPSC-CM) wiederherzustellen, die der Wirkung des Arzneimittelkandidaten ausgesetzt waren, indem eine Simulation mit dem fortgeschrittenen mathematischen Modell der Kardiomyozyten-Elektrophysiologie mit zuvor optimierten Parametern und den prozentualen Anteilen der Hemmung ausgeführt wird, die in dem vorangegangenen Schritt berechnet wurden oder im Voraus aus früheren Experimenten bekannt sind;

Schritt 8 - Wiederherstellen des modifizierten extern stimulierten Aktionspotentials (AP) in seiner ursprünglichen Form, die vor der Applikation des Arzneimittelkandidaten aufgezeichnet wurde, indem die Datei des Restaurationsstrom-Stimulus unter Anwendung eines Dynamic-Clamp-Protokolls auf denselben humanen, von induzierten pluripotenten Stammzellen abgeleiteten Kardiomyozyten (hiPSC-CM) angewendet wird, auf dem der Arzneimittelkandidat appliziert wurde;

Schritt 9 - Verwenden der berechneten prozentualen Anteile der Hemmung, um die Metriken zu berechnen, die für das proarrhythmogene Risiko des Arzneimittelkandidaten prädiktiv sind, wie beispielsweise die über ein Aktionspotential integrierte Nettoladung, die durch die Summe mehrerer nach innen und außen gerichteter Ionenströme übertragen wird.

2. Verfahren nach Anspruch 1, wobei das β-Escin perforierte Ganzzell-Patch-Clamp Verfahren auf dem einen oder den mehreren humanen, von induzierten pluripotenten Stammzellen abgeleiteten Kardiomyozyten (hiPSC-CM) angewendet wird, um die Form des anfänglichen, extern stimulierten Aktionspotentials (AP) und die anfänglichen Peak- oder stationären Amplituden der mindestens fünf Ionenströme über eine Zeitdauer von 10 bis 20 Minuten unter Kontrollbedingungen zu stabilisieren, was das "Run-Down"-Phänomen vermeidet, das von zunehmendem Verkürzen der Form des anfänglichen, extern stimulierten Aktionspotentials (AP) begleitet wird, das in der klassischen Anwendung des Patch-Clamp mit ruptierter Zellmembran an der einen oder den mehreren humanen induzierten pluripotenten, von Stammzellen derivierten Cardiomyozyten (hiPSC-CM) in Erscheinung tritt.

3. Verfahren nach Anspruch 1 oder 2, wobei das Berechnen von Parametern, einschließend Ionenleitfähigkeits-Oberflächendichten und Skalierungsfaktoren in Bezug auf das fortgeschrittene mathematische Modell der Kardiomyozyten-Elektrophysiologie, wie beispielsweise das bevorzugte modifizierte Modell O'Hara-Rudy2011 von mindestens fünf menschlichen ventrikulären Ionenströmen in den Reihen von Spannungs-Klemme-Protokollen ausgeführt werden kann, indem physiologische externe bzw. extrazelluläre und interne bzw. Pipetten-Lösungen und in Abwesenheit von spezifischen Blockern bei Messung von Peak- oder stationären Amplituden der mindestens fünf Ionenströme verwendet werden, einschließlich mindestens

- Peak des schnellen spannungsabhängigen, nach innen gerichteten Na$^+$-Strom ($I_{Na}$) mit Skalierungsfaktor der Leitfähigkeitsdichte scgna
- Peak des L-Typ, nach innen gerichteten Ca$^{2+}$-Strom ($I_{CaL}$) mit Skalierungsfaktor der Leitfähigkeitsdichte scgcal
- nach außen gerichteter K$^+$- Übergangsstrom ($I_{to}$) mit Skalierungsfaktor der Leitfähigkeitsdichte scgto
- "Funny" durch Hyperpolarisation aktivierter Strom ($I_f$) mit Skalierungsfaktor der Leitfähigkeitsdichte scgf
- der Summe der schnellen ($I_{Kr}$) und langsamen ($I_{Ks}$) Komponenten des verzögerten Gleichrichter-K$^+$-Stroms, wobei jedoch gewöhnlich $I_{Kr}$ unter Kontrollbedingungen die dominierende Komponente ist, mit Skalierungsfaktor der Leitfähigkeitsdichte scgkr.

4. Verfahren nach einem der vorhergehenden Ansprüche, *wobei* die Optimierung von einem oder mehreren Parametern des fortgeschrittenen mathematischen Modells der Kardiomyozyten-Elektrophysiologie, um die Form des anfänglichen Aktionspotentials (AP) exakt zu reproduzieren, wie beispielsweise Skalierungsfaktoren der Leitfähigkeits-Oberflächendichten und Temperaturen, in einer sequentiellen, parallelen oder kombinierten Weise auf der Hochleistungscomputereinheit (HPCU) ausgeführt wird, indem die Summe der zum Quadrat genommenen Differenzen zwischen dem experimentell aufgezeichneten anfänglichen extern stimulierten Aktionspotential (AP) und der modellgenerierten Form des extern stimulierten anfänglichen Aktionspotentials (AP) als Optimalitätskriterium genommen werden und einige oder alle der zuvor berechneten Skalierungsfaktoren verwendet werden.

5. Verfahren nach einem der vorhergehenden Ansprüche, *wobei* die Datei des Restaurationsstrom-Stimulus, die die Summe aller Ionenströme darstellt, die durch den Arzneimittelkandidaten gehemmt werden, wie sie beispielsweise durch das fortgeschrittene mathematische Modell der Kardiomyozyten-Elektrophysiologie erzeugt wird, als ein Kriterium zur Validierung für die Detektion des proarrhythmogenen Risikos des Arzneimittelkandidaten verwendet werden kann.

## Revendications

1. Procédé pour la détection *in vitro* du risque pro-arythmogène d'un médicament candidat dans lequel, par l'utilisation d'une plateforme patch-clamp automatisée (APCP) haute performance capable de réaliser des mesures et des enregistrements expérimentaux d'au moins cinq courants ioniques ventriculaires humains et d'un potentiel d'action cadencée en externe (AP) sur des cardiomyocytes issus de cellules souches pluripotentes induites humaines (hiPSC-CM) et par l'utilisation d'un algorithme informatisé fonctionnant dans une unité informatique haute performance (HPCU) pour une optimisation rapide de paramètres d'un modèle mathématique d'électrophysiologie de cardiomyocytes avancée pour reproduire précisément la forme dudit potentiel d'action cadencée en externe (AP), la séquence d'étapes en une seule opération suivante est réalisée sur lesdits cardiomyocytes issus de cellules souches pluripotentes induites humaines (hiPSC-CM):

Etape 1 - Capture sur une puce microfluidique d'un ou de plusieurs cardiomyocytes issus de cellules souches pluripotentes induites humaines (hiPSC-CM) à partir d'une suspension, formation de scellements giga, suivie par l'obtention d'une configuration de cellules entières par une perforation de β-aescine, qui permet de stabiliser la forme du potentiel d'action cadencée en externe (AP) sur une durée généralement entre 10 et 20 minutes;

Etape 2 - Séparation, enregistrement et mesure d'amplitudes de crête ou à l'état stable des au moins cinq courants ioniques par l'application sur lesdits un ou plusieurs cardiomyocytes issus de cellules souches pluripotentes induites humaines (hiPSC-CM) approchés dans le patch-clamp de cellules entières perforées par la β-aescine d'une série de protocoles de verrouillage de tension bien définis;

Etape 3 - Calcul sur la base d'une analyse automatisée d'enregistrements en utilisant la série desdits protocoles de verrouillage de tension de densités de surface de conductance ionique pour chacun desdits au moins cinq courants ioniques et de facteurs d'échelle pour les densités de surface de conductance ionique de chacun desdits au moins cinq courants ioniques relativement à des valeurs par défaut dudit modèle mathématique d'électrophysiologie de cardiomyocytes avancée;

Etape 4 - Mesure et enregistrement d'un potentiel d'action cadencée en externe (AP) initial desdits un ou plusieurs cardiomyocytes issus de cellules souches pluripotentes induites humaines (hiPSC-CM) par l'application d'un protocole de verrouillage de courant standard avec un seul balayage et avec des stimuli de courants prédéterminés.

Etape 5

- 5a - Application dudit médicament candidat à une concentration donnée sur un ou plusieurs cardiomyocytes issus de cellules souches pluripotentes induites humaines (hiPSC-CM) qui remplissent 2 conditions, à savoir avoir un potentiel de repos < -50 mV et avoir un potentiel d'action cadencée en externe (AP) initial de type ventriculaire avec un rapport de durée de potentiel d'action APD50/APD90 > 70%, ladite application du médicament candidat étant pendant une période de temps prédéterminée, de préférence de 8 à 15 minutes et plus préférablement de 10 minutes, et simultanément, durant la même période de temps prédéterminée:

- 5b - Mesure et enregistrement du potentiel d'action cadencée en externe (AP) modifié sous l'effet dudit médicament candidat par l'application du même protocole de verrouillage de courant que pour la détermination du potentiel d'action cadencée en externe (AP) initial, périodiquement à des intervalles de temps fixés, de préférence une minute, et détection des effets pro-arythmogènes dudit médicament candidat sur la forme du potentiel d'action cadencée en externe (AP) initial, tels que des dépolarisations ultérieures précoces ou retardées;

- 5c - Optimisation rapide de paramètres dudit modèle mathématique d'électrophysiologie de cardiomyocytes avancée en utilisant lesdits facteurs d'échelle pour les densités de surface de conductance ionique de chacun desdits au moins cinq courants ioniques dans le but de reproduire précisément la forme dudit potentiel d'action cadencée en externe (AP) initial sur des cardiomyocytes issus de cellules souches pluripotentes induites humaines (hiPSC-CM) ayant comme résultat une forme générée par un modèle dudit potentiel d'action cadencée en externe (AP) initial;

Etape 6 - Mesure et enregistrement des amplitudes des au moins cinq courants ioniques inhibés après l'application dudit médicament candidat en utilisant la même série de protocoles de verrouillage de tension que dans l'étape 2, en les comparant aux amplitudes initiales en l'absence dudit médicament candidat et calcul des pourcentages d'inhibition pour chacun d'eux;

Etape 7 - Génération d'un fichier de stimuli de courant de restauration représentant la somme de tous les courants ioniques inhibés par le médicament candidat avec un signe inversé et des stimuli prédéterminés initiaux, pour qu'il soit utilisé en outre pour restaurer la forme du potentiel d'action cadencée en externe (AP) initial dans les un ou plusieurs cardiomyocytes issus de cellules souches pluripotentes induites humaines (hiPSC-CM) soumis à l'action du médicament candidat en effectuant une simulation avec ledit modèle mathématique d'électrophysiologie de cardiomyocytes avancée avec les paramètres optimisés précédemment et les pourcentages d'inhibition calculés dans l'étape précédente ou connus à l'avance à partir d'expériences antérieures;

Etape 8 - Restauration du potentiel d'action cadencée en externe (AP) modifié à sa forme initiale, enregistrée avant l'application dudit médicament candidat par l'application du fichier de stimuli de courant de restauration en utilisant un protocole de verrouillage dynamique sur le même cardiomyocyte issu de cellules souches pluripotentes induites humaines (hiPSC-CM) que celui sur lequel le médicament candidat a été appliqué;

Etape 9 - Utilisation des pourcentages d'inhibition calculés pour calculer des paramètres prédisant le risque pro-arythmogène dudit médicament candidat tels que la charge nette intégrée sur un potentiel d'action portée par la somme de plusieurs courants ioniques intérieurs et extérieurs.

**2.** Procédé selon la revendication 1, dans lequel un patch-clamp de cellules entières perforées par la β-aescine sur les un ou plusieurs cardiomyocytes issus de cellules souches pluripotentes induites humaines (hiPSC-CM) est utilisé dans le but de stabiliser la forme du potentiel d'action cadencée en externe (AP) initial et les amplitudes de crête et à l'état stable initiales desdits au moins cinq courants ioniques sur un intervalle de temps de 10-20 min. dans des conditions de contrôle, ce qui prévient le phénomène de diminution accompagné par un raccourcissement progressif de ladite forme de potentiel d'action cadencée en externe (AP) initial qui se produit dans l'approche de patch-clamp de cellules entières « rompue » classique sur lesdits un ou plusieurs cardiomyocytes issus de cellules souches pluripotentes induites humaines (hiPSC-CM).

**3.** Procédé selon les revendications 1 et 2, dans lequel le calcul de paramètres incluant les densités de surface de conductance ionique et les facteurs d'échelle relativement au modèle mathématique d'électrophysiologie de cardiomyocytes avancée, tel que de préférence le modèle O'Hara-Rudy2011 modifié, d'au moins cinq courants ioniques ventriculaires humains peut être atteint dans la série de protocoles de verrouillage de tension en utilisant des solutions de pipette physiologiques externes, respectivement extracellulaires, et internes, respectivement et en l'absence de bloqueurs spécifiques, lors d'une mesure d'amplitudes de crête ou à l'état stable desdits au moins cinq courants ioniques, incluant au moins :

- un courant de Na$^+$ intérieur dépendant de la tension rapide de crête ($I_{Na}$) avec un facteur d'échelle de densité de conductance scgna
- un courant de Ca$^{2+}$ intérieur de type L de crête ($I_{CaL}$) avec un facteur d'échelle de densité de conductance scgcal
- un courant de K$^+$ extérieur transitoire ($I_{to}$) avec un facteur d'échelle de densité de conductance scgto
- un courant activé par hyperpolarisation « drôle » ($I_f$) avec un facteur d'échelle de densité de conductance scgf
- une somme des composantes rapides ($I_{Kr}$) et lentes ($I_{Ks}$) du courant de K$^+$ redresseur retardé, mais où habituellement $I_{Kr}$ est la composante dominante dans les conditions de contrôle, avec un facteur d'échelle de densité de conductance scgkr.

**4.** Procédé selon l'une quelconque des revendications précédentes, dans lequel l'optimisation d'un ou de plusieurs paramètres dudit modèle mathématique d'électrophysiologie de cardiomyocytes avancé pour reproduire précisément la forme dudit potentiel d'action (AP) initial, tel que par exemple des facteurs d'échelle de densités de surface de conductance et de température, est effectuée d'une manière séquentielle, parallèle ou combinée dans l'unité informatique haute performance (HPCU), en prenant comme critère d'optimalité la somme de différences au carré entre le potentiel d'action cadencée en externe (AP) initial enregistré expérimentalement et la forme générée par un modèle dudit potentiel d'action cadencée en externe (AP) initial et en utilisant une partie ou la totalité des facteurs d'échelle calculés antérieurement.

**5.** Procédé selon l'une quelconque des revendications précédentes, dans lequel le fichier de stimuli de courant de restauration, représentant la somme de tous les courant ioniques inhibés par ledit médicament candidat, tel que généré par ledit modèle mathématique d'électrophysiologie de cardiomyocytes avancée peut être utilisé comme un critère de validation pour la détection du risque pro-arythmogène dudit médicament candidat.

FIGURE 1

**Original CiPA algorithm (Sager PT et al. Am Heart J 2014 167(3):292-300)**

① | ② | ③

| test of drug effects on multiple cell lines with heterologous expression of human cardiac ion channels | use of experimental data obtained in step 1 with an advanced cardiomyocyte electrophysiology mathematical model (O'Hara-Rudy 2011) to predict proarrhythmogenic effects (EADs, DADs, etc.) | validation of model predictions via experiments on human iPSC-derived cardiomyocytes |

FIGURE 1.A

Fig. 1B

FIGURE 2

FIGURE 3

FIGURE 4

FIGURE 5 (TABLE 1)

## Improvements to O'Hara-Rudy2011 model (cpp script) to adequately reproduce AP recordings on human pluripotent stem cell-derived cardiomyocytes via parameter optimization

➤ Setting cell type =2 (mid-myocardial cardiomyocyte)

➤ Running with fixed time step (5 $\mu$s), output to file at 1 ms intervals

➤ Setting 3 cycles of 3000 ms

➤ Setting 3 stimuli of 0.5 ms 2 nA; first stimulus at 500 ms

➤ Introduction of 4-state circular Markov model for $I_f$ (Männikkö R *et al.* 2005), with redefined rates to observe experimental $\tau_{act}$ at -120 mV (avg.1350±660 ms)

➤ Redefining cell geometry to start from given $C_m$ and compute cylinder length $l$ for default radius a = 0.0011 cm

➤ Setting capacitive/geometric area ratio rgc=1.2 (instead of default rgc=2)

➤ Setting scaling factors for conductance densities, $Ca^{2+}$ release/uptake

➤ Including temperature coefficients $Q_{10}$ for main ion current gating time constants (from own experimental data)

```
void RGC()
{
...
double tm=pow(1.98,-(T-310)/10)/(6.765*exp((v+11.64)/34.77)
+8.552*exp((v+77.42)/5.955)); //orig tm=1.0/...

...
double thf=pow(1.19,-(T-310)/10)/(1.432e-5*exp(-(v+1.196)/6.285)+6.149*
exp((v+0.5096)/20.27)); //orig thf=1.0/...
double ths=pow(1.19,-(T-310)/10)/(0.009794*exp(-(v+17.95)/28.05)
+0.3343*exp((v+5.730)/56.66)); //orig ths=1.0/...

...
double tj=pow(1.19,-(T-310)/10)*(2.038+1.0/(0.02136*exp(-(v+100.6)
/8.281)+0.3052*exp((v+0.9941)/38.45))); //orig tj=...

...
double thL=pow(1.19,-(T-310)/10)*200.0; //orig thL=200.0

...
double tiF=pow(1.72,-(T-310)/10)*(4.562+1/(0.3933*exp((-(v+100.0))
/100.0)+0.08004*exp((v+50.0)/16.59))); //orig tiF=4.562+...
double tiS=pow(1.72,-(T-310)/10)*(23.62+1/(0.001416*exp((-(v+96.52))
/59.05)+1.780e-8*exp((v+114.1)/8.079))); //orig tiS=23.62+...

...
double td=pow(3.18,-(T-310)/10)*(0.6+1.0/(exp(-0.05*(v+6.0))
+exp(0.09*(v+14.0)))); //orig td=0.6+...

...
```

FIGURE 5 (TABLE 1) CONTINUED

```
double tff=pow(3.18,-(T-310)/10)*(7.0+1.0/(0.0045*exp(-(v+20.0)/10.0)
+0.0045*exp((v+20.0)/10.0)));  //orig tff=7.0+...
double tfs=pow(3.18,-(T-310)/10)*(1000.0+1.0/(0.000035*exp(-(v+5.0)
/4.0)+0.000035*exp((v+5.0)/6.0)));  //orig tfs=1000.0+...

double tfcaf=pow(3.18,-(T-310)/10)*(7.0+1.0/(0.04*exp(-(v-4.0)/7.0)
+0.04*exp((v-4.0)/7.0)));  //orig tfcaf=7.0+...
double tfcas=pow(3.18,-(T-310)/10)*(100.0+1.0/(0.00012*exp(-v/3.0)
+0.00012*exp(v/7.0)));  //orig tfcas=100.0+...
...
double tjca=pow(3.18,-(T-310)/10)*75.0;  //orig tjca=75.0;
...
double txrf=pow(3.95,(310-T)/10)*(12.98+1.0/(0.3652*exp((v-31.66)
/3.869)+4.123e-5*exp((-(v-47.78))/20.38)));  //orig txrf=12.98+...
double txrs=pow(3.95,(310-T)/10)*(1.865+1.0/(0.06629*exp((v-34.70)
/7.355)+1.128e-5*exp((-(v-29.74))/25.94)));  //orig txrs=1.865+...
...
double txs1=pow(1.51,(310-T)/10)*(817.3+1.0/(2.326e-4*exp((v+48.28)
/17.80)+0.001292*exp((-(v+210.0))/230.0)));  //orig txs1=817.3+...
...
double txs2=pow(1.51,(310-T)/10)*1.0/(0.01*exp((v-50.0)/20.0)+0.0193*
exp((-(v+66.54))/31.0));  //orig txs2=1.0/...
...
double txk1=pow(1.26,(310-T)/10)*122.2/(exp((-(v+127.2))/20.36)
+exp((v+236.8)/69.33));  //orig txk1=122.2/...
...
INaCa_i=pow(2.5,(T-310)/10)*0.8*Gncx*allo*(zna*JncxNa+zca*JncxCa);  //Q10 2.5 for NCX1
(Elias CL, Xue XH, Marshall CR, Omelchenko A, Hryshko LV, Tibbits GF. Temperature
dependence of cloned mammalian and salmonid cardiac Na(+)/Ca(2+) exchanger isoforms. Am
J Physiol Cell Physiol. 2001 Sep;281(3):C993-C1000)

...
INaCa_ss=pow(2.5,(T-310)/10)*0.2*Gncx*allo*(zna*JncxNa+zca*JncxCa);  //Q10 2.5 for NCX1
(Elias CL, Xue XH, Marshall CR, Omelchenko A, Hryshko LV, Tibbits GF. Temperature
dependence of cloned mammalian and salmonid cardiac Na(+)/Ca(2+) exchanger isoforms. Am
J Physiol Cell Physiol. 2001 Sep;281(3):C993-C1000)

...
        af = pow(3.0,(T-298)/10)*0.18*exp(-F*(v+65-dvy)/(R*T));//orig. (v + 64.0)...*5*exp(...If
opening rate Q10 3 from Pena F et al. 2006
        bf = pow(3.0,(T-298)/10)*0.18*exp(F*(v+65-dvy)/(R*T));//orig ...*5*exp(...If closing rate
Q10 3 from Pena F et al. 2006

...
}
```

## FIGURE 6

FIGURE 7

FIGURE 8 (TABLE 2)

| Strong effect on AP shape | Weak effect on AP shape |
|---|---|
| scgcal | scgna |
| scgkr | scgks |
| scgf | scgtos |
| scgki | scgpca |
| scgncx | scgrel |
| scgnak | scgup |
| scgtof | scgcab |
| scgkb | scgnab |

FIGURE 9

FIGURE 10

Initial estimates of scaling factors, $C_m$, and v
scgna, scgcal, scgf, scgkr, scgtof
scgki 0.3, scgncx 0.05, scgnak 3.0, T=300
All other scaling factors 1

**First round: scgcal and scgkr**
30-40 values for each between 0 and 2
T= 298, 299, 300, 301, 302
scgna & scgtof initial, scgf 0.3, scgki 0.3, scgncx 0.05,
scgnak 3.0
All other scaling factors 1; 10 cycles (output last 3 cyc.)

Select optimal parameter set (smallest summed squared differences with experimental samples)

**Second round: scgf, scgki, scgnak at selected T**
20 values for scgf & scgki between 0 and 0.6
4 values for scgnak (0 to 3) and scgncx (0 to 1)
scgna & scgtof initial
All other scaling factors 1; 10 cycles (output last 3 cyc.)

Select optimal parameter set (smallest summed squared differences with experimental samples)

**Third round: reestimate scgf & scgki, estimate scgtof & scgkb at selected T**
10 values for scgf & scgki between ±0.2 of previous est.
10 values for scgkb (0 to 2) and 7 for scgtof (0 to 7)
scgna initial
All other scaling factors 1; 10 cycles (output last 3 cyc.)

Select optimal parameter set (smallest summed squared differences with experimental samples)

**Fourth round: reestimate scgcal, scgkr, scgncx, scgnak, scgf at two T (selected and +/- 1 K depending on APD90)**
5 values for scgcal, scgkr, scgf between±0.1 of prev. est.
5 values for scgncx, scgnak between±0.2 of prev. est.
All other scaling factors 1; 10 cycles (output last 3 cyc.)

Select optimal parameter set (smallest summed squared differences with experimental samples)

## FIGURE 11

**Exp. UNIVBUCURESTI_2017-11-30__11.26.19_Ch1**

**Exp. C10_2018-01-24__15.29.42_Ch2**

**Exp. C10_2018-01-25__10.10.11_Ch2**

**Exp. C10_2018-01-25__10.47.46_Ch2**

FIGURE 12 (TABLE 3)

| Rate of change over approximately 10 min. | Patch-clamp approach | |
|---|---|---|
| Ion current peak/steady amplitude | Ruptured ($n = 4$) Mean ± SD | β-escin perf. ($n = 9\text{-}12$) Mean ± SD |
| $I_{Na}$ (%change/min) | -2.63 ± 0.92 | 2.80 ± 3.92 |
| $I_{CaL}$ (%change/min) | -2.74 ± 1.50 | -0.37 ± 3.76 |
| $I_{to}$ (%change/min) | 4.29 ± 3.88 | -0.31 ± 1.04 |
| $I_{Kr}$ (%change/min) | 9.59 ± 17.28 | 1.91 ± 5.05 |
| $I_f$ (%change/min) | 0.83 ± 1.53 | 2.00 ± 3.70 |
| AP parameter | Ruptured ($n = 3$) Mean ± SD | β-escin perf. ($n = 4$) mean±SD |
| APD90 decay rate (%change/min) | 4.6 ± 1.1 | 0.7 ± 1.6 |

FIGURE 13

FIGURE 14

Exp. C10_2018-08-24__14.21.16

initial
nifedipine 1 μM
restoration of $I_{CaL}$ by dynamic clamp

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030153067 A1
- US 2013153067 A1

### Non-patent literature cited in the description

- *International Conference on Harmonization of Technical Requirements for Registration of Pharmaceuticals for Human Use,* 2005
- *Comprehensive in vitro Proarrhythmia Assay,* 23 July 2013
- **SCHEEL et al.** Action potential Characterization of Human Induced Pluripotent Stem Cell-Derived Cardiomyocytes Using Automated patch-Clamp Tehnology. *Assay and Drug Development Technologies,* October 2014, vol. 12 (8), 457-469
- **S. POLAK ; M. K. PUGSLEY ; N. STOCKBRIDGE ; C. GARNETT ; B. WISNIOWSKA.** *AAPS J,* 2015, vol. 17, 1025-32
- **B. AMUZESCU ; O. SCHEEL ; T. KNOTT.** *J Phys Chem Biophys.,* 2014, vol. 4, 1-4
- **D. NOBLE.** *J Pharmacol Sci.,* 2008, vol. 107, 107-17
- **J. KRAMER ; C. A. OBEJERO-PAZ ; G. MYATT ; Y. A. KURYSHEV ; A. BRUENING-WRIGHT ; J. S. VERDUCCI ; A. M. BROWN.** *Sci Rep,* 2013, 3
- **P. T. SAGER ; G. GINTANT ; J. R. TURNER ; S. PETTIT ; N. STOCKBRIDGE.** *Am Heart J,* 2014, vol. 167, 292-300
- **T. O'HARA ; L. VIRAG ; A. VARRÓ ; Y. RUDY.** *PLoS Comput Biol,* 2011, vol. 7, 26
- **R. WALLIS ; C. BENSON ; B. DARPO ; G. GINTANT ; Y. KANDA ; K. PRASAD ; D. G. STRAUSS ; J. P. VALENTIN.** *J Pharmacol Toxicol Methods,* 2018, vol. 8719, 30626-9
- **H. HUANG ; M. K. PUGSLEY ; B. FERMINI ; M. J. CURTIS ; J. KOERNER ; M. ACCARDI ; S. AUTHIER.** *J Pharmacol Toxicol Methods,* 2017, vol. 87, 11-23
- **M. J. WINDLEY ; N. ABI-GERGES ; B. FERMINI ; J. C. HANCOX ; J. I. VANDENBERG ; A. P. HILL.** *J Pharmacol Toxicol Methods,* 2017, vol. 87, 99-107
- **Z. LI ; S. DUTTA ; J. SHENG ; P. N. TRAN ; W. WU ; K. CHANG ; T. MDLULI ; D. G. STRAUSS ; T. COLATSKY.** *Circ Arrhythm Electrophysiol,* 2017, vol. 10, 004628
- **Z. LI ; S. DUTTA ; J. SHENG ; P. N. TRAN ; W. WU ; T. COLATSKY.** *J Pharmacol Toxicol Methods,* 2016, vol. 81, 233-9
- **S. DUTTA ; K. C. CHANG ; K. A. BEATTIE ; J. SHENG ; P. N. TRAN ; W. W. WU ; M. WU ; D. G. STRAUSS ; T. COLATSKY ; Z. LI.** *Front,* 2017, vol. 8, 616
- **K. H. GILCHRIST ; G. F. LEWIS ; E. A. GAY ; K. L. SELLGREN ; S. GREGO.** *Toxicol Appl Pharmacol,* 2015, vol. 288, 249-57
- **K. HARRIS ; M. AYLOTT ; Y. CUI ; J. B. LOUTTIT ; N. C. MCMAHON ; A. SRIDHAR.** *Toxicol Sci,* 2013, vol. 134, 412-26
- **A. MEHTA ; Y. CHUNG ; G. L. SEQUIERA ; P. WONG ; R. LIEW ; W. SHIM.** *Toxicol Sci,* 2013, vol. 131, 458-69
- **P. MULDER ; T. DE KORTE ; E. DRAGICEVIC ; U. KRAUSHAAR ; R. PRINTEMPS ; M. L. H. VLAMING ; S. R. BRAAM ; J. P. VALENTIN.** *J Pharmacol Toxicol Methods,* 2018, vol. 91, 36-42
- **C. T. BOT ; K. JUHASZ ; F. HAEUSERMANN ; L. POLONCHUK ; M. TRAEBERT ; S. STOELZLE-FEIX.** *J Pharmacol Toxicol Methods,* 2018, vol. 8719, 30600-2
- **H. M. HIMMEL.** *J Pharmacol Toxicol Methods,* 2013, vol. 68, 97-111
- **N. HU ; T. WANG ; H. WAN ; L. ZHUANG ; R. KETTENHOFEN ; X. ZHANG ; Y. S. ZHANG ; W. XU ; M. GOSSMANN ; H. BOHLEN.** *Biosens Bioelectron,* 2018, vol. 117, 354-365
- **B. KOCI ; G. LUERMAN ; A. DUENBOSTELL ; R. KETTENHOFEN ; H. BOHLEN ; L. COYLE ; B. KNIGHT ; W. KU ; W. VOLBERG ; J. R. WOSKA, JR.** *Toxicol Appl Pharmacol,* 2017, vol. 329, 121-127
- **X. ZHANG ; L. GUO ; H. ZENG ; S. L. WHITE ; M. FURNISS ; B. BALASUBRAMANIAN ; E. LIS ; A. LAGRUTTA ; F. SANNAJUST ; L. L. ZHAO.** *J Pharmacol Toxicol Methods,* 2016, vol. 81, 201-16
- **S. BEDUT ; C. SEMINATORE-NOLE ; V. LAMAMY ; S. CAIGNARD ; J. A. BOUTIN ; O. NOSJEAN ; J. P. STEPHAN ; F. COGE.** *Am J Physiol Heart Circ Physiol,* 2016, vol. 311, 3

- **K. BLINOVA ; J. STOHLMAN ; J. VICENTE ; D. CHAN ; L. JOHANNESEN ; M. P. HORTIGON-VINAGRE ; V. ZAMORA ; G. SMITH ; W. J. CRUMB ; L. PANG.** *Toxicol Sci,* 2017, vol. 155, 234-247
- **P. W. BURRIDGE ; S. THOMPSON ; M. A. MILLROD ; S. WEINBERG ; X. YUAN ; A. PETERS ; V. MAHAIRAKI ; V. E. KOLIATSOS ; L. TUNG ; E. T. ZAMBIDIS.** *PLoS One,* 2011, vol. 6, 0018293
- **M. P. HORTIGON-VINAGRE ; V. ZAMORA ; F. L. BURTON ; J. GREEN ; G. A. GINTANT ; G. L. SMITH.** *Toxicol Sci,* 2016, vol. 154, 320-331
- **A. LOPEZ-IZQUIERDO ; M. WARREN ; M. RIEDEL ; S. CHO ; S. LAI ; R. L. LUX ; K. W. SPITZER ; I. J. BENJAMIN ; M. TRISTANI-FIROUZI ; C. J. JOU.** *Am J Physiol Heart Circ Physiol,* 2014, vol. 307, 29
- **E. R. PFEIFFER ; R. VEGA ; P. M. MCDONOUGH ; J. H. PRICE ; R. WHITTAKER.** *J Pharmacol Toxicol Methods,* 2016, vol. 81, 263-73
- **H. ZENG ; M. I. ROMAN ; E. LIS ; A. LAGRUTTA ; F. SANNAJUST.** *J Pharmacol Toxicol Methods,* 2016, vol. 81, 217-22
- **W. R. GILES ; D. NOBLE.** *Biophys J,* 2016, vol. 110, 278-80
- **Y. KURATA ; I. HISATOME ; S. IMANISHI ; T. SHIBAMOTO.** *Am J Physiol Heart Circ Physiol.,* 2002, vol. 283, H2074-101
- **O. SCHEEL ; S. FRECH ; B. AMUZESCU ; J. EISFELD ; K. H. LIN ; T. KNOTT.** *Assay Drug Dev Technol,* 2014, vol. 12, 457-69
- **R. MÄNNIKKÖ ; S. PANDEY ; H. P. LARSSON ; F. ELINDER.** *J Gen Physiol.,* 2005, vol. 125, 305-26
- **T. R. SHANNON ; F. WANG ; J. PUGLISI ; C. WEBER ; D. M. BERS.** *Biophys J.,* 2004, vol. 87, 3351-71